# EUROPEAN PATENT SPECIFICATION

(11) **EP 3 730 612 B1**
(45) Date of publication and mention of the grant of the patent: **04.02.2026**
(21) Application number: 18892953.3
(22) Date of filing: 19.12.2018
(51) Int. Cl.: C12N 15/09, A61K 35/17, A61P 35/00, C07K 14/52, C07K 14/54, C07K 14/705, C07K 19/00, C12N 5/0783

(54) **IMMUNOCOMPETENT CELL THAT EXPRESSES A CELL SURFACE MOLECULE SPECIFICALLY RECOGNIZING HUMAN MESOTHELIN, IL-7 AND CCL19**
IMMUNKOMPETENTE ZELLE, DIE EIN ZELLOBERFLÄCHENMOLEKÜL EXPRIMIERT, DAS SPEZIFISCH HUMANES MESOTHELIN, IL-7 UND CCL19 ERKENNT
CELLULE IMMUNOCOMPÉTENTE EXPRIMANT UNE MOLÉCULE DE SURFACE CELLULAIRE RECONNAISSANT SPÉCIFIQUEMENT LA MÉSOTHÉLINE HUMAINE, IL-7 ET CCL19

(30) Priority: 24.12.2017 JP 2017247109
(43) Date of publication of application: 28.10.2020
(73) Proprietor: Noile-Immune Biotech, Inc., Tokyo 105-0012 (JP)
(72) Inventor: TAMADA, Koji, Ube-shi, Yamaguchi 755-8505 (JP); SAKODA, Yukimi, Ube-shi, Yamaguchi 755-8505 (JP); ADACHI, Keishi, Ube-shi, Yamaguchi 755-8505 (JP)
(74) Representative: Dennemeyer & Associates S.A.
(86) International application number: PCT/JP2018/046888
(87) International publication number: WO 2019/124468

(56) References cited:
- EP-A1- 3 205 720
- EP-A1- 3 431 597
- WO-A1-2008/143684
- WO-A1-2009/068204
- WO-A1-2016/056228
- WO-A1-2017/159736
- WO-A2-2013/063419
- JP-A- 2011 504 372
- MORELLO A. ET AL.: "Mesothelin-targeted CARs: driving T cells to solid tumors", CANCER DISCOV., vol. 6, no. 2, 2016, pages 133 - 146, XP055239486, DOI: doi:10.1158/2159-8290.CD-15-0583
- ADACHI K ET AL.: "IL -7 and CCL19 expression in CAR-T cells improves immune cell infiltration and CAR-T cell survival in the tumor", NAT. BIOTECHNOL., vol. 36, no. 4, April 2018 (2018-04-01), pages 346 - 351, XP055474269, DOI: doi:10.1038/nbt.4086

## Description

### Technical Field

The present invention relates to an immunocompetent cell that expresses a cell surface molecule specifically recognizing human mesothelin, interleukin 7 (IL-7), and chemokine (C-C motif) ligand 19 (CCL19), a pharmaceutical composition comprising the immunocompetent cell, an expression vector comprising a nucleic acid encoding a cell surface molecule specifically recognizing mesothelin, a nucleic acid encoding IL-7, and a nucleic acid encoding CCL19, and a method for producing an immunocompetent cell that expresses a cell surface molecule specifically recognizing human mesothelin, IL-7, and CCL19, comprising introducing the expression vector to an immunocompetent cell.

### Background Art

Malignant tumors are diseases that affect many people in the world and in general, are widely treated by chemotherapy, radiotherapy, or surgical therapy. However, there have been various problems such as the occurrence of adverse reactions, a loss of some functions, and recurrence or metastasis which cannot be treated. Accordingly, the development of immune cell therapy has been advanced in recent years in order to maintain higher quality of life (QOL) of patients. This immune cell therapy is a therapy which involves collecting immunocompetent cells from a patient, performing procedures to enhance the immune functions of the immunocompetent cells, amplifying the cells, and bringing the cells back to the patient. Specifically, a therapy of collecting T cells from a patient, introducing a nucleic acid encoding chimeric antigen receptor (hereinafter, also referred to as "CAR") to the T cells, and bringing the T cells back to the patient (see Non-patent Document 1) is known. This therapy is currently under clinical trial worldwide and has produced results indicating efficacy on, for example, malignant hematopoietic organ tumors such as leukemia or lymphoma.

Meanwhile, the present inventors have proposed immune cell therapy of markedly suppressing solid cancer by co-expressing IL-7 and CCL19 (see Patent Documents 1 and 2). This method can enhance the activation of endogenous immunocompetent cells or their ability to accumulate on tumor cells.

Mesothelin is known to be expressed in cells of cancer such as mesothelioma, colorectal cancer (rectum cancer and colon cancer), pancreatic cancer, ovary cancer, lung cancer, breast cancer, or head and neck cancer. CAR-T cells targeting the mesothelin are disclosed (see Patent Documents 3 and 4).

### Prior Art Documents

### Patent Documents

Patent Document 1: International Publication No. WO 2016/056228
Patent Document 2: International Publication No. WO 2017/159736
Patent Document 3: U.S. Patent Application Publication No. 2014/0301993
Patent Document 4: Japanese unexamined Patent Application Publication No. 2017-518053

### Non-patent Documents

Non-patent Document 1: Yozo Nakazawa, The Shinshu Medical Journal 61 (4): 197-203 (2013)

### Summary of the Invention

### Object to be Solved by the Invention

The development of techniques that can be adapted to solid cancer found to receive no sufficient therapeutic effects of conventional immune cell therapy are underway by developing immunocompetent cell therapy using CAR-expressing T cells, TCR-expressing T cells, or the like that co-express IL-7 and CCL19, as described above, and markedly improving the ability of immunocompetent cells to proliferate, the ability of immunocompetent cells to survive, or the ability of host's immunocompetent cells to accumulate. On the other hand, the development of CAR-expressing immunocompetent cells targeting mesothelin highly expressed in cancer (e.g., mesothelioma and pancreatic cancer) cells has not yet produced satisfactory clinical results due to insufficient local accumulation of immunocompetent cells on cancer, and a risk of recurrence of tumor ascribable to short exertion of antitumor effects, etc. Accordingly, an object of the present invention is to provide a novel immunocompetent cell targeting mesothelin.

### Means to Solve the Object

The present inventors have studied the further possibility of our own previously developed T cells that express CAR, IL-7 and CCL19. As a result, the present inventors have completed the present invention by finding that CAR containing single chain antibody specifically binding to human mesothelin and containing a particular amino acid sequence specifically recognizing human mesothelin as a cell surface molecule can be selectively used to exert cytotoxic activity against cancer cells expressing mesothelin and to suppress reduction in survival rate caused by tumor formed by the cancer cells expressing mesothelin.

Specifically, the present invention is as follows:
[1] An immunocompetent cell that comprises: (a) an exogenous nucleic acid encoding a chimeric antigen receptor (CAR) specifically recognizing human mesothelin; (b) an exogenous nucleic acid encoding interleukin 7 (IL-7); and (c) an exogenous nucleic acid encoding chemokine (C-C motif) ligand 19 (CCL19),
   the CAR comprising a single chain antibody, a transmembrane region, and a signaling region that induces activation of the immunocompetent cell,
   wherein the single chain antibody in the CAR is (1-1) a single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18.
[2] The immunocompetent cell according to [1], wherein the immunocompetent cell is an immunocompetent cell separated from a bodily fluid.
[3] The immunocompetent cell according to [1], wherein the exogenous nucleic acid encoding IL-7, and the exogenous nucleic acid encoding CCL19 are an artificially synthesized nucleic acid encoding human IL-7, and an artificially synthesized nucleic acid encoding human CCL19.
[4] The immunocompetent cell according to [3], wherein the exogenous nucleic acid encoding a CAR specifically recognizing human mesothelin, the exogenous nucleic acid encoding IL-7, and the exogenous nucleic acid encoding CCL19 are integrated into the genome.
[5] The immunocompetent cell according to any one of [1] to [4], wherein the single chain antibody in the CAR is (1-3) a single chain antibody comprising a heavy chain variable region consisting of the amino acid sequence shown by SEQ ID NO: 1, and a light chain variable region consisting of the amino acid sequence shown by SEQ ID NO: 2.
[6] The immunocompetent cell according to any one of [1] to [5], wherein the transmembrane region in the CAR comprises an amino acid sequence shown by SEQ ID NO: 7.
[7] The immunocompetent cell according to any one of [1] to [6], wherein the signaling region that induces the activation of the immunocompetent cell in the CAR comprises at least one member selected from intracellular regions of polypeptides of CD28, 4-1BB (CD137), GITR, CD27, OX40, HVEM, CD3ζ, and Fc receptor-associated γ chain.
[8] The immunocompetent cell according to any one of [1] to [6], wherein the signaling region that induces the activation of the immunocompetent cell in the CAR comprises the amino acid sequences shown by SEQ ID NOs: 8, 9 and 10.
[9] The immunocompetent cell according to any one of [1] to [8], wherein the heavy chain variable region and the light chain variable region are connected via a peptide linker consisting of a 2- to 30-amino acid sequence.
[10] The immunocompetent cell according to [9], wherein the peptide linker consists of the amino acid sequence shown by SEQ ID NO: 26 or SEQ ID NO: 27.
[11] The immunocompetent cell according to any one of [1] to [10], wherein the signaling region that induces the activation of the immunocompetent cell in the CAR comprises a polypeptide of a CD28 intracellular region, a polypeptide of a 4-1BB intracellular region, and a polypeptide of a CD3ζ intracellular region.
[12] The immunocompetent cell according to any one of [1] to [11], wherein the immunocompetent cell is a T cell.
[13] The immunocompetent cell according to any one of [1] to [12], wherein the immunocompetent cell is a T cell separated from a bodily fluid.
[14] A pharmaceutical composition comprising an immunocompetent cell according to any one of [1] to [13] and a pharmaceutically acceptable additive.
[15] The pharmaceutical composition according to [14] for use in the treatment of cancer.
[16] An expression vector comprising a nucleic acid encoding a chimeric antigen receptor (CAR) specifically recognizing human mesothelin, a nucleic acid encoding IL-7, and a nucleic acid encoding CCL19, the CAR comprising a single chain antibody, a transmembrane region, and a signaling region that induces activation of an immunocompetent cell, wherein the single chain antibody of the CAR is (1-1) a single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18.
[17] A method for producing an immunocompetent cell that expresses a chimeric antigen receptor (CAR) specifically recognizing human mesothelin, IL-7, and CCL19, comprising introducing the expression vector of [16] to an immunocompetent cell.

### Effect of the Invention

The immunocompetent cell of the present invention has cytotoxic activity against cancer cells expressing human mesothelin and is capable of suppressing the formation of tumor expressing human mesothelin. Also, the immunocompetent cell of the present invention has suppressive effects on the recurrence of cancer cells.

### Brief Description of Drawings

[Figure 1] Figure 1 is a diagram showing the arrangements (a) and amino acid sequences (b) of 9 types of anti-human mesothelin scFvs produced in Example 1.
[Figure 2] Figure 2 is a diagram showing results of examining the expression of CAR in anti-human mesothelin CAR-IL-7/CCL19-expressing T cells in Example 2. Figure 2(a) shows results about CAR, IL-7, and CCL19 non-expressing T cells (Non-infection), and Figures 2(b) to 2(d) show results about anti-human mesothelin CAR-IL-7/CCL19-expressing T cells having VH07(15)VL07 (signal peptide T), VH07(15)VL07 (signal peptide P), and VH36(15)VL36, respectively, as a scFv region.
[Figure 3] Figure 3 is a diagram showing results of examining the expression of CAR in anti-human mesothelin CAR-IL-7/CCL19-expressing T cells in Example 2. Figure 3(a) shows results about CAR, IL-7, and CCL19 non-expressing T cells (Non-infection), and Figures 3(b) to 3(e) show results about anti-human mesothelin CAR-IL-7/CCL19-expressing T cells having VH07(15)VL07, VL07(15)VH07, VH07(25)VL07, and VL07 (25) VH07, respectively, as a scFv region.
[Figure 4] Figure 4 is a diagram confirming the expression level of mesothelin in tumor cell lines using a flow cytometer in Example 3.
[Figure 5] Figure 5 is an illustrative diagram of a co-culture test on anti-human mesothelin CAR-IL-7/CCL19-expressing T cells and a mesothelin-positive tumor cell line or a mesothelin-negative tumor cell line in Example 4.
[Figure 6A] Figure 6A is a diagram showing results of measuring a surviving tumor cell line ACC-MESO-1 by flow cytometry in Example 4.
[Figure 6B] Figure 6B is a diagram showing results of measuring a surviving tumor cell line NCI-H2052 by flow cytometry in Example 4.
[Figure 6C] Figure 6C is a diagram showing results of measuring a surviving tumor cell line A498 by flow cytometry in Example 4.
[Figure 7A] Figure 7A is a diagram showing results of measuring produced IFN-γ after co-culture of anti-human mesothelin CAR-IL-7/CCL19-expressing T cells and a mesothelin-positive tumor cell line ACC-MESO-1 in Example 4.
[Figure 7B] Figure 7B is a diagram showing results of measuring produced IFN-γ after co-culture of anti-human mesothelin CAR-IL-7/CCL19-expressing T cells and a mesothelin-positive tumor cell line NCI-H2052 in Example 4.
[Figure 7C] Figure 7C is a diagram showing results of measuring produced IFN-γ after co-culture of anti-human mesothelin CAR-IL-7/CCL19-expressing T cells and a mesothelin-negative tumor cell line A498 in Example 4.
[Figure 8] Figure 8 is a diagram showing results of measuring surviving leukocytes (Lymphocyte number) or PAN02 tumor cell line by flow cytometry in Example 5.
[Figure 9] Figure 9 is a diagram showing results of measuring produced IFN-γ after co-culture of anti-mesothelin CAR-IL-7/CCL19-expressing T cells and a PAN02 tumor cell line in Example 5.
[Figure 10] Figure 10 is a diagram showing results of measuring survival rates by the administration of anti-mesothelin CAR-mouse IL-7/mouse CCL19-expressing mouse T cells to tumor model mice in Example 6.
[Figure 11] Figure 11 is a diagram showing results of measuring tumor volumes by the administration of anti-mesothelin CAR-mouse IL-7/mouse CCL19-expressing mouse T cells to tumor model mice in Example 6.
[Figure 12A] Figure 12A is a diagram showing results of photographing mice on days 1, 3, 7, 10, 14, 21, 31, and 38 for an exposure time of 30 seconds in Example 7 when the day on which ACC-MESO-1-GFP-Luc was administered was defined as day 1.
[Figure 12B] Figure 12B is a diagram showing results of photographing mice on days 45, 59, 73, 87, 101, 115, 129, and 143 for an exposure time of 30 seconds in Example 7 when the day on which ACC-MESO-1-GFP-Luc was administered was defined as day 1. The photograph on day 129 of the second individual counted from the left is omitted because the individual died.
[Figure 13] Figure 13 is a graph showing the relationship between the number of days from administration and a mouse survival rate in Example 7.
[Figure 14] Figure 14 is a graph showing the relationship between the number of days from administration and the total quantity of fluorescence in Example 7.

### Mode of Carrying Out the Invention

The immunocompetent cell of the present invention can be any immunocompetent cell that comprises: (a) an exogenous nucleic acid encoding a chimeric antigen receptor (CAR) specifically recognizing human mesothelin; (b) an exogenous nucleic acid encoding interleukin 7 (IL-7); and (c) an exogenous nucleic acid encoding chemokine (C-C motif) ligand 19 (CCL19), the CAR comprising a single chain antibody, a transmembrane region, and a signaling region that induces activation of the immunocompetent cell, wherein the single chain antibody in the CAR is (1-1) a single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18. This immunocompetent cell is capable of suppressing tumor formation ascribable to cancer cells expressing human mesothelin.

### (Human mesothelin)

Human mesothelin, a 40 kDa protein, is rarely expressed in normal cells and highly expressed in cancer (e.g., mesothelioma and pancreatic cancer) cells. Sequence information on human mesothelin can be appropriately obtained by the search of a publicly known document or a database such as NCBI (http://www.ncbi.nlm.nih.gov/guide/). Examples of the amino acid sequence information on human mesothelin can include GenBank accession No. NP_037536.2, AAV87530.1, and their isoforms.

### (Cell surface molecule)

According to the invention, the cell surface molecule specifically recognizing human mesothelin is a CAR specifically recognizing human mesothelin. The CAR is an artificial chimeric protein in which a single chain antibody (scFv) recognizing a cell surface antigen on cancer cells is fused with a signaling region that induces the activation of T cells.

The cell surface molecule is preferably localized on the cell surface of the immunocompetent cell through a signal peptide (leader sequence). Examples of the signal peptide can include polypeptides of an immune globulin heavy chain, an immunoglobulin light chain, CD8, T cell receptor α and β chains, CD3ζ, CD28, CD3ε, CD45, CD4, CD5, CD8, CD9, CD16, CD22, CD33, CD37, CD64, CD80, CD86, CD134, CD137, ICOS, CD154, and a GITR-derived signal peptide (leader sequence). Specific examples thereof can include a polypeptide that consists of an amino acid sequence having 85% or higher, preferably 90% or higher, more preferably 95% or higher, further preferably 98% or higher sequence identity to the amino acid sequence shown by SEQ ID NO: 11 or 12, and has action equivalent to that of the amino acid sequence of SEQ ID NO: 11 or 12, and a polypeptide that consists of an amino acid sequence derived from the amino acid sequence shown by SEQ ID NO: 11 or 12 by the deletion, substitution, insertion, and/or addition of one or several amino acids, and has action equivalent to that of the amino acid sequence shown by SEQ ID NO: 11 or 12. The signal peptide has been removed in a mature protein after the completion of localization.

In the present specification, the "amino acid sequence derived by the deletion, substitution, insertion, and/or addition of one or several amino acid residues" encompasses even an amino acid sequence with amino acid residues deleted, substituted, inserted, and/or added, for example, within the range of 1 to 30 residues, preferably within the range of 1 to 20 residues, more preferably within the range of 1 to 15 residues, further preferably within the range of 1 to 10 residues, further preferably within the range of 1 to 5 residues, further preferably within the range of 1 to 3 residues, further preferably within the range of 1 to 2 residues. These variation treatments of the amino acid residues can be performed by an arbitrary method known to those skilled in the art, such as chemical synthesis, a genetic engineering approach, or mutagenesis.

In the present specification, the term "identity" means the degree of polypeptide or polynucleotide sequence similarity (which is determined by the matching between a query sequence and another sequence (nucleic acid or protein sequence), preferably of the same type thereas). Preferred examples of the computer program method for calculating and determining the "identity" can include GCG BLAST (Basic Local Alignment Search Tool) (Altschul et al., J. Mol. Biol. 1990, 215: 403-410; Altschul et al., Nucleic Acids Res. 1997, 25: 3389-3402; and Devereux et al., Nucleic Acid Res. 1984, 12: 387), BLASTN 2.0 (Gish W., http://blast.Wustl.edu,1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; and Needleman and Wunsch, J. Mol. Biol. 1970, 48: 443-453).

### (Single chain antibody specifically recognizing human mesothelin)

The cell surface molecule is CAR, and a single chain antibody (scFv) specifically recognizing human mesothelin is contained as a molecule specifically recognizing human mesothelin. In the single chain antibody specifically recognizing human mesothelin, the heavy chain variable region (VH) and the light chain variable region (VL) of an antibody specifically recognizing human mesothelin can be connected through a peptide linker for linking the heavy chain variable region and the light chain variable region. The combination of the heavy chain variable region and the light chain variable region in the single chain antibody specifically recognizing human mesothelin can is the combination given below. The light chain variable region may be positioned upstream (on the N-terminal side) or downstream (on the C-terminal side) of the heavy chain variable region.

(1-1) A combination of a heavy chain variable region comprising heavy chain CDR (complementarity determining region) 1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18.

An example of the combination of the heavy chain variable region and the light chain variable region in the single chain antibody specifically recognizing human mesothelin is the following combination:
(1-3) a combination of a heavy chain variable region consisting of the amino acid sequence shown by SEQ ID NO: 1, and a light chain variable region consisting of the amino acid sequence shown by SEQ ID NO: 2.

### (Peptide linker)

The heavy chain variable region and the light chain variable region can be connected via a peptide linker. The length of the peptide linker is 2 to 30, preferably 15 to 25, more preferably 15, or 25. Specifically, preferred examples thereof can include a polypeptide that consists of an amino acid sequence having 85% or higher, preferably 90% or higher, more preferably 95% or higher, further preferably 98% or higher sequence identity to the amino acid sequence shown by SEQ ID NO: 26 or 27 comprising a glycine-serine continuous sequence, and has action equivalent to that of the amino acid sequence of SEQ ID NO: 26 or 27, and a polypeptide that consists of an amino acid sequence derived from the amino acid sequence shown by SEQ ID NO: 26 or 27 by the deletion, substitution, insertion, and/or addition of one or several amino acids, and has action equivalent to that of the amino acid sequence shown by SEQ ID NO: 26 or 27.

### (IL-7 and CCL19)

The IL-7 is a cytokine essential for the survival of T cells and is produced by non-hematopoietic cells such as stromal cells of the bone marrow, the thymus gland, or a lymphoid organ or tissue. On the other hand, T cells themselves are rarely found to have the ability to produce the IL-7.

The CCL19 is mainly produced from dendritic cells or macrophages of lymph nodes and has a function of initiating the migration of T cells, B cells, or mature dendritic cells via its receptor CCR7.

The organism from which the IL-7 or the CCL19 is derived is not particularly limited and is preferably a human. The amino acid sequences of these proteins are available from a publicly known sequence database such as GenBank. Examples of the amino acid sequence of human IL-7 can include a sequence registered under GenBank accession No: NM_000880.3 (SEQ ID NO: 28) and its isoform. Examples of the amino acid sequence of human CCL19 can include a sequence registered under GenBank accession No: NM_006274.2 (SEQ ID NO: 29) and its isoform. Although the IL-7 and the CCL19 may have a signal peptide, the signal peptide is removed in a mature protein. For example, in the amino acid sequence of human IL-7 described in SEQ ID NO: 28, a sequence from positions 1 to 25 corresponds to the signal peptide. For example, in the amino acid sequence of human CCL19 described in SEQ ID NO: 29, a sequence from positions 1 to 21 corresponds to the signal peptide.

The IL-7 or the CCL19 may be a variant of the natural protein as described above. Examples of the IL-7 variant can include a polypeptide that consists of an amino acid sequence having 85% or higher, preferably 90% or higher, more preferably 95% or higher, further preferably 98% or higher sequence identity to the amino acid sequence of human IL-7 described in SEQ ID NO: 28, and has action of enhancing a cell proliferation rate or a cell survival rate by IL-7, and a polypeptide that consists of an amino acid sequence derived from the amino acid sequence of human IL-7 described in SEQ ID NO: 28 by the deletion, substitution, insertion, and/or addition of one or several amino acids, and has action of enhancing a cell proliferation rate or a cell survival rate by IL-7. Examples of the human CCL19 variant can include a polypeptide that consists of an amino acid sequence having 85% or higher, preferably 90% or higher, more preferably 95% or higher, further preferably 98% or higher sequence identity to the amino acid sequence of human CCL19 described in SEQ ID NO: 29, and has the cell migrating action of CCL19, and a polypeptide that consists of an amino acid sequence derived from the amino acid sequence of human CCL19 described in SEQ ID NO: 29 by the deletion, substitution, insertion, and/or addition of one or several amino acids, and has the cell migrating action of CCL19.

### (Additional immune function control factor)

The immunocompetent cell of the present invention may further express an additional immune function control factor such as IL-15, CCL21, IL-2, IL-4, IL-12, IL-13, IL-17, IL-18, IP-10, interferon-γ, MIP-1alpha, GM-CSF, M-CSF, TGF-beta, or TNF-alpha. The additional immune function control factor is preferably an immune function control factor other than IL-12.

### (Transmembrane region)

Examples of the transmembrane region according to the present invention can include polypeptides of transmembrane regions derived from CD8, T cell receptor α and β chains, CD3ζ, CD28, CD3ε, CD45, CD4, CD5, CD8, CD9, CD16, CD22, CD33, CD37, CD64, CD80, CD86, CD134, CD137, ICOS, CD154, and GITR. Preferred examples thereof can include a polypeptide that comprises an amino acid sequence having 85% or higher, preferably 90% or higher, more preferably 95% or higher, further preferably 98% or higher sequence identity to the amino acid sequence of a human CD8 transmembrane region shown by SEQ ID NO: 7, and has action equivalent to that of the amino acid sequence shown by SEQ ID NO: 7, and a polypeptide that consists of an amino acid sequence derived from the amino acid sequence shown by SEQ ID NO: 7 by the deletion, substitution, insertion, and/or addition of one or several amino acids, and has action equivalent to that of the amino acid sequence shown by SEQ ID NO: 7. CAR is anchored to the cell membranes of T cells through such a transmembrane region.

The transmembrane region may comprise a hinge region that consists of an arbitrary oligopeptide or polypeptide and has a length of 1 to 100 amino acids, preferably 10 to 70 amino acids. Examples of the hinge region can include the hinge region of human CD8.

### (Immunocompetent cell activating signaling region)

The immunocompetent cell activating signaling region is a region capable of transducing signals into the cell when the cell surface molecule recognizes mesothelin. The immunocompetent cell activating signaling region preferably comprises at least one or more members selected from intracellular regions of polypeptides of CD28, 4-1BB (CD137), GITR, CD27, OX40, HVEM, CD3ζ, or Fc receptor-associated γ chain, and more preferably three polypeptides, i.e., a polypeptide of a CD28 intracellular region, a polypeptide of a 4-1BB intracellular region, and a polypeptide of a CD3ζ intracellular region. Examples of the amino acid sequence of the CD28 intracellular region can include a polypeptide that comprises an amino acid sequence having 85% or higher, preferably 90% or higher, more preferably 95% or higher, further preferably 98% or higher sequence identity to the amino acid sequence shown by SEQ ID NO: 8, and has action equivalent to that of the amino acid sequence shown by SEQ ID NO: 8, and a polypeptide that consists of an amino acid sequence derived from the amino acid sequence shown by SEQ ID NO: 8 by the deletion, substitution, insertion, and/or addition of one or several amino acids, and has action equivalent to that of the amino acid sequence shown by SEQ ID NO: 8. Examples of the amino acid sequence of the 4-1BB intracellular region can include a polypeptide that comprises an amino acid sequence having 85% or higher, preferably 90% or higher, more preferably 95% or higher, further preferably 98% or higher sequence identity to the amino acid sequence shown by SEQ ID NO: 9, and has action equivalent to that of the amino acid sequence shown by SEQ ID NO: 9, and a polypeptide that consists of an amino acid sequence derived from the amino acid sequence shown by SEQ ID NO: 9 by the deletion, substitution, insertion, and/or addition of one or several amino acids, and has action equivalent to that of the amino acid sequence shown by SEQ ID NO: 9. Examples of the amino acid sequence of the CD3ζ intracellular region can include a polypeptide that comprises an amino acid sequence having 85% or higher, preferably 90% or higher, more preferably 95% or higher, further preferably 98% or higher sequence identity to the amino acid sequence shown by SEQ ID NO: 10, and has action equivalent to that of the amino acid sequence shown by SEQ ID NO: 10, and a polypeptide that consists of an amino acid sequence derived from the amino acid sequence shown by SEQ ID NO: 10 by the deletion, substitution, insertion, and/or addition of one or several amino acids, and has action equivalent to that of the amino acid sequence shown by SEQ ID NO: 10. When a T cell is used as the immunocompetent cell, a polypeptide capable of transducing signals into the T cell can be selected. When other immunocompetent cells are used, polypeptides capable of transducing signals into the immunocompetent cells can be selected. In the case of using a T cell as the immunocompetent cell, examples of the immunocompetent cell activating signaling region can include a polypeptide comprising the amino acid sequences shown by SEQ ID NOs: 8, 9 and 10 and can preferably include a polypeptide comprising the amino acid sequences shown by SEQ ID NOs: 8, 9 and 10 in order from the N-terminal side.

### (Extracellular hinge region and spacer)

An extracellular hinge region consisting of an arbitrary oligopeptide or polypeptide may be located between the cell surface molecule recognizing mesothelin and the transmembrane region. Examples of the length of the extracellular hinge region can include 1 to 100 amino acid residues, preferably 10 to 70 amino acid residues. Examples of such an extracellular hinge region can include hinge regions derived from CD8, CD28, and CD4, and an immune globulin hinge region.

A spacer region consisting of an arbitrary oligopeptide or polypeptide may be located between the transmembrane region and the immunocompetent cell activating signaling region. Examples of the length of the spacer region can include 1 to 100 amino acid residues, preferably 10 to 50 amino acid residues. Examples of such a spacer region can include a glycine-serine continuous sequence.

### (Arrangement of each region)

In the CAR, each region described above can be arranged in order of the single chain antibody, the transmembrane region, and the immunocompetent cell activating signaling region from the N terminus. Specific examples thereof can include CAR in which the single chain antibody specifically recognizing human mesothelin, the extracellular hinge region of human CD8, the transmembrane region of human CD8, the T cell activating signaling region of human CD28, the T cell activating signaling region of human 4-1BB, and the T cell activating signaling region of human CD3ζ are arranged in order from the N-terminal side.

### (Protein to be expressed by suicide gene)

The immunocompetent cell of the present invention may express a protein having a function of killing its own cell, such as herpes simplex virus thymidine kinase (HSV-TK) or inducible caspase 9 (protein to be expressed by a suicide gene) . The expression of such a protein based on the suicide gene directly or secondarily induces a substance having cellular toxicity and can confer the function of killing its own cell. Hence, for example, the immunocompetent cell of the present invention when present in a living body can be controlled by the administration of a drug activating the function after disappearance of tumor according to the course of treatment of cancer. Specifically, a risk of cytokine release syndrome can be reliably reduced, if necessary, for the immunocompetent cell of the present invention.

Examples of the drug activating the function of herpes simplex virus thymidine kinase (HSV-TK) or inducible caspase 9 can include ganciclovir for the former and AP1903 which is chemical induction of dimerization (CID) for the latter (Cooper LJ., et al., Cytotherapy. 2006; 8 (2): 105-17; Jensen M. C. et al., Biol Blood Marrow Transplant. 2010 Sep; 16 (9): 1245-56; Jones BS. Front Pharmacol. 2014 Nov 27; 5: 254; Minagawa K., Pharmaceuticals (Basel). 2015 May 8; 8 (2): 230-49; and Bole-Richard E., Front Pharmacol. 2015 Aug 25; 6: 174).

### (Type of cell for immunocompetent cell)

The type of the cell for the immunocompetent cell is not particularly limited as long as the cell is involved in immune response and can express the cell surface molecule specifically recognizing human mesothelin, the IL-7 and the CCL19 by the introduction of a nucleic acid encoding the cell surface molecule specifically recognizing human mesothelin, a nucleic acid encoding the IL-7, and a nucleic acid encoding the CCL19. The cell is preferably an immunocompetent cell separated from bodily fluid. Examples thereof can include a lymphoid cell such as a T cell, a natural killer cell (NK cell), and a B cell, an antigen presenting cell such as a monocyte, a macrophage, and a dendritic cell, and a granulocyte such as a neutrophil, an eosinophil, a basophil, and a mast cell, separated from bodily fluid. Specifically, preferred examples thereof can include a T cell derived or separated from a bodily fluid of a mammal such as a human, a dog, a cat, a pig, or a mouse, preferably a T cell derived or separated from a bodily fluid of a human. The T cell derived from a bodily fluid of a mammal such as a human, a dog, a cat, a pig, or a mouse includes a T cell obtained by artificially culturing *ex vivo* a T cell separated (collected) from the bodily fluid of a mammal such as a human, a dog, a cat, a pig, or a mouse, or a T cell subcultured from this T cell. The separated T cell can be a cell population mainly comprising T cells. Such a cell population may comprise additional cells other than T cells and preferably comprises T cells at a proportion of 50% or higher, preferably 60% or higher, more preferably 70% or higher, further preferably 80% or higher, most preferably 90% or higher. The T cell can be obtained by separating a cell population comprising the immunocompetent cell from a body fluid such as blood or bone marrow fluid, a tissue such as a spleen tissue, the thymus gland, or a lymph node, or immunocompetent cells infiltrating a cancer tissue such as primary tumor, metastatic tumor, or cancerous ascites. In order to elevate the proportion of T cells comprised in the cell population, the T cell may be further isolated or purified, if necessary, from the separated cell population by a standard method. A cell produced from an ES cell or an iPS cell may be utilized for the immunocompetent cell. Examples of such a T cell can include an alpha-beta T cell, a gamma-delta T cell, a CD8⁺ T cell, a CD4⁺ T cell, a tumor infiltrating T cell, a memory T cell, a naive T cell, and a NKT cell. The origin of the immunocompetent cell may be the same as or different from an administration subject. When the administration subject is a human, an autologous cell collected from a patient himself or herself as the administration subject may be used as the immunocompetent cell, or an allogeneic cell collected from another person may be used thereas. Specifically, the donor and the recipient may or may not be the same and are preferably the same.

### (Method for producing immunocompetent cell)

Examples of the method for producing the immunocompetent cell of the present invention can include a production method of introducing the expression vector of the present invention mentioned later to an immunocompetent cell by a method described in, for example, Patent Document 1 or 2. Alternative examples, which are not encompassed by the wording of the claims but which may be considered as useful for understanding the invention, can include a method of purifying and obtaining an immunocompetent cell from a transgenic mammal produced by implanting a vector for expression of a cell surface molecule specifically recognizing human mesothelin, IL-7, and/or CCL19 into a fertilized egg, and a production method of further introducing, if necessary, the vector for expression of a cell surface molecule specifically recognizing human mesothelin, IL-7, and/or CCL19 to the immunocompetent cell purified and obtained from the transgenic mammal.

In the case of introducing a nucleic acid encoding a cell surface molecule, a nucleic acid encoding IL-7, and a nucleic acid encoding CCL19, or the vector of the present invention mentioned later to an immunocompetent cell, the method for introducing the nucleic acids or the vector can be any method for introducing the nucleic acids or the vector to the immunocompetent cell. Examples thereof can include a method such as an electroporation method (Cytotechnology, 3, 133 (1990)), a calcium phosphate method (Japanese unexamined Patent Application Publication No. 2-227075), a lipofection method (Proc. Natl. Acad. Sci. U.S.A., 84, 7413 (1987)), and a viral infection method. Examples of such a viral infection method can include a method of transfecting a packaging cell such as a GP2-293 cell (manufactured by Takara Bio Inc.), a Plat-GP cell (manufactured by Cosmo Bio Co., Ltd.), a PG13 cell (ATCC CRL-10686), or a PA317 cell (ATCC CRL-9078) with the vector to be introduced and a packaging plasmid to produce a recombinant virus, and infecting the immunocompetent cell with the recombinant virus (Patent Document 2).

In the case of producing the "immunocompetent cell of the present invention" using an expression vector, the immunocompetent cell can be produced by any of the following methods:
(1) a method of introducing a vector for expression of a CAR specifically recognizing human mesothelin, IL-7 and CCL19, containing a nucleic acid encoding the CAR specifically recognizing human mesothelin, a nucleic acid encoding the IL-7 and a nucleic acid encoding the CCL19 to an immunocompetent cell;
(2) a method of introducing, at the same time or in stages, two types of vectors, i.e., a vector for expression of a CAR specifically recognizing human mesothelin, containing a nucleic acid encoding the CAR specifically recognizing human mesothelin, and a vector for expression of IL-7 and CCL19, containing a nucleic acid encoding the IL-7 and a nucleic acid encoding the CCL19, to an immunocompetent cell;
(3) a method of introducing, at the same time or in stages, two types of vectors, i.e., a vector for expression of a CAR specifically recognizing human mesothelin and IL-7, containing a nucleic acid encoding the CAR specifically recognizing human mesothelin and a nucleic acid encoding the IL-7, and a vector for expression of CCL19, containing a nucleic acid encoding the CCL19, to an immunocompetent cell;
(4) a method of introducing, at the same time or in stages, two types of vectors, i.e., a vector for expression of a CAR specifically recognizing human mesothelin and CCL19, containing a nucleic acid encoding the CAR specifically recognizing human mesothelin and a nucleic acid encoding the CCL19, and a vector for expression of IL-7, containing a nucleic acid encoding the IL-7, to an immunocompetent cell;
(5) a method of introducing, at the same time or in stages, two types of vectors, i.e., a vector for expression of a CAR specifically recognizing human mesothelin and IL-7, containing a nucleic acid encoding the CAR specifically recognizing human mesothelin and a nucleic acid encoding the IL-7, and a vector for expression of a CAR specifically recognizing human mesothelin and CCL19, containing a nucleic acid encoding the CAR specifically recognizing human mesothelin and a nucleic acid encoding the CCL19, to an immunocompetent cell;
(6) a method of introducing, at the same time or in stages, two types of vectors, i.e., a vector for expression of a CAR specifically recognizing human mesothelin and IL-7, containing a nucleic acid encoding the CAR specifically recognizing human mesothelin and a nucleic acid encoding the IL-7, and a vector for expression of IL-7 and CCL19, containing a nucleic acid encoding the IL-7 and a nucleic acid encoding the CCL19, to an immunocompetent cell;
(7) a method of introducing, at the same time or in stages, two types of vectors, i.e., a vector for expression of a CAR specifically recognizing human mesothelin and CCL19, containing a nucleic acid encoding the CAR specifically recognizing human mesothelin and a nucleic acid encoding the CCL19, and a vector for expression of IL-7 and CCL19, containing a nucleic acid encoding the IL-7 and a nucleic acid encoding the CCL19, to an immunocompetent cell; and
(8) a method of introducing, at the same time or in stages, three types of vectors, i.e., a vector for expression of a CAR specifically recognizing human mesothelin, containing a nucleic acid encoding the CAR specifically recognizing human mesothelin, a vector for expression of IL-7, containing a nucleic acid encoding the IL-7, and a vector for expression of CCL19, containing a nucleic acid encoding the CCL19, to an immunocompetent cell.

In the case of producing the "immunocompetent cell of the present invention" using an expression vector, an immunocompetent cell that expresses the CAR specifically recognizing human mesothelin is prepared in advance, and the immunocompetent cell may be produced by any of the following methods using this immunocompetent cell that expresses the CAR specifically recognizing human mesothelin:
(1) a method of introducing a vector for expression of IL-7 and CCL19, containing a nucleic acid encoding the IL-7 and a nucleic acid encoding the CCL19 to the immunocompetent cell that expresses the CAR specifically recognizing human mesothelin; and
(2) a method of introducing, at the same time or in stages, two types of vectors, i.e., a vector for expression of IL-7, containing a nucleic acid encoding the IL-7, and a vector for expression of CCL19, containing a nucleic acid encoding the CCL19, to the immunocompetent cell that expresses the CAR specifically recognizing human mesothelin.

In the case of using each of the immunocompetent cells described above, cultures of the immunocompetent cell, containing the immunocompetent cell may be used. The nucleic acid encoding the CAR specifically recognizing human mesothelin, the nucleic acid encoding the IL-7, and the nucleic acid encoding the CCL19 may be integrated in the genome of the immunocompetent cell or may not be integrated in the genome (e.g., episomally), for use. In the case of using each of the immunocompetent cells described above, a mixture of an immunocompetent cell in which the nucleic acid encoding the CAR specifically recognizing human mesothelin, the nucleic acid encoding the IL-7, and the nucleic acid encoding the CCL19 are integrated in the genome of the immunocompetent cell, and an immunocompetent cell in which the nucleic acid encoding the CAR specifically recognizing human mesothelin, the nucleic acid encoding the IL-7, and the nucleic acid encoding the CCL19 are not integrated in the genome, may be used.

The "immunocompetent cell of the present invention" as described above may be produced by integrating a nucleic acid encoding the CAR specifically recognizing human mesothelin, a nucleic acid encoding the IL-7, and a nucleic acid encoding the CCL19 in the genome of a cell so as to be expressible under the control of a suitable promoter by use of a publicly known gene editing technique. Examples of the publicly known gene editing technique include a technique using an endonuclease such as zing finger nuclease, TALEN (transcription activator-like effector nuclease), CRISPR (clustered regularly interspaced short palindromic repeat)-Cas system. In the case of allowing an immunocompetent cell that expresses, for example, CAR specifically recognizing human mesothelin (anti-human mesothelin CAR) to express an additional exogenous protein, a polynucleotide comprising a nucleotide sequence encoding the additional exogenous protein may similarly be integrated in the genome of the cell so as to be expressible under the control of a suitable promoter by use of the gene editing technique. Specific examples of such a method include: a method of integrating a polynucleotide comprising a nucleotide sequence encoding anti-human mesothelin CAR (or an additional protein), functionally linked to a suitable promoter to a non-coding region or the like of the cell genome; and a method of integrating a polynucleotide comprising a nucleotide sequence encoding anti-human mesothelin CAR (or an additional protein) to downstream of an endogenous promoter of the cell genome. Examples of the endogenous promoter include TCRα and TCRβ promoters.

### (Administration subject)

For the avoidance of doubt, any references to methods of treatment by therapy or surgery or *in vivo* diagnosis in this description are to be interpreted as references to the immunocompetent cell of the present invention for use in those methods. Examples of the administration subject can include a mammal and a mammalian cell. Examples of such a mammal can include a human, a mouse, a dog, a rat, a guinea pig, a rabbit, a bird, sheep, a pig, cattle, a horse, a cat, a monkey, and a chimpanzee, optionally a human.

### (Expression vector)

The expression vector of the present invention comprises a nucleic acid encoding a chimeric antigen receptor (CAR) specifically recognizing human mesothelin, a nucleic acid encoding IL-7, and a nucleic acid encoding CCL19, the CAR comprising a single chain antibody, a transmembrane region, and a signaling region that induces activation of an immunocompetent cell,
wherein the single chain antibody of the CAR is (1-1) a single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18. Those skilled in the art are capable of designing and producing an expression vector that permits expression of the desired protein (polypeptide) in immunocompetent cells. The expression vector is, hereinafter, also referred to as an "IL-7/CCL19 expression-anti-human mesothelin vector".

The IL-7/CCL19 expression-anti-human mesothelin vector may further contain a nucleic acid encoding an additional immune function control factor such as IL-15, CCL21, IL-2, IL-4, IL-12, IL-13, IL-17, IL-18, IP-10, interferon-γ, MIP-1alpha, GM-CSF, M-CSF, TGF-β, TNF-α, or a checkpoint inhibiting antibody or its fragment. The nucleic acid encoding an additional immune function control factor is preferably a nucleic acid encoding an immune function control factor other than IL-12.

### (Nucleic acid)

In the present specification, the "nucleic acid" can be any molecule of polymerized nucleotides and/or molecules having functions equivalent to those of the nucleotides. Examples thereof can include RNA which is a polymer of ribonucleotides, DNA which is a polymer of deoxyribonucleotides, a mixed polymer of ribonucleotides and deoxyribonucleotides, and a nucleotide polymer comprising a nucleotide analog. Alternatively, a nucleotide polymer comprising a nucleic acid derivative may be used. The nucleic acid may be a single-stranded nucleic acid or a double-stranded nucleic acid. The double-stranded nucleic acid also includes a double-stranded nucleic acid in which one of the strands hybridizes under stringent conditions to the other strand.

The nucleotide analog can be any molecule as long as the molecule is a ribonucleotide, a deoxyribonucleotide, RNA or DNA modified in order to improve or stabilize nuclease resistance, in order to enhance affinity for a complementary strand nucleic acid, in order to enhance cell permeability, or in order to visualize the molecule, as compared with RNA or DNA. The nucleotide analog may be a naturally occurring molecule or a non-natural molecule. Examples thereof include a nucleotide analog with a modified sugar moiety and a nucleotide analog with a modified phosphodiester bond.

The nucleotide analog with a modified sugar moiety can be any molecule as long as an arbitrary chemical structural substance is added to or replaced for a portion or the whole of the chemical structure of a sugar in a nucleotide. Specific examples thereof include a nucleotide analog substituted by 2'-O-methyl ribose, a nucleotide analog substituted by 2'-O-propyl ribose, a nucleotide analog substituted by 2'-methoxyethoxy ribose, a nucleotide analog substituted by 2'-O-methoxyethyl ribose, a nucleotide analog substituted by 2'-O-[2-(guanidium)ethyl]ribose, a nucleotide analog substituted by 2'-fluoro ribose, bridged nucleic acid (BNA) having two cyclic structures by the introduction of a bridged structure to the sugar moiety, more specifically, locked nucleic acid (LNA) with an oxygen atom at position 2' and a carbon atom at position 4' bridged via methylene, and ethylene bridged nucleic acid (ENA) [Nucleic Acid Research, 32, e175 (2004)], and can further include peptide nucleic acid (PNA) [Acc. Chem. Res., 32, 624 (1999)], oxypeptide nucleic acid (OPNA) [J. Am. Chem. Soc., 123, 4653 (2001)], and peptide ribonucleic acid (PRNA) [J. Am. Chem. Soc., 122, 6900 (2000)].

The nucleotide analog with a modified phosphodiester bond can be any molecule as long as an arbitrary chemical structural substance is added to or replaced for a portion or the whole of the chemical structure of a phosphodiester bond in a nucleotide. Specific examples thereof can include a nucleotide analog substituted by a phosphorothioate bond, and a nucleotide analog substituted by a N3'-P5' phosphoramidate bond [Cell Engineering, 16, 1463-1473 (1997)] [RNAi Method and Antisense Method, Kodansha Ltd. (2005)].

The nucleic acid derivative can be any molecule as long as the molecule is a nucleic acid with another chemical substance added thereto in order to improve or stabilize nuclease resistance, in order to enhance affinity for a complementary strand nucleic acid, in order to enhance cell permeability, or in order to visualize the molecule, as compared with a nucleic acid. Specific examples thereof can include a 5'-polyamine-added derivative, a cholesterol-added derivative, a steroid-added derivative, a bile acid-added derivative, a vitamin-added derivative, a Cy5-added derivative, a Cy3-added derivative, a 6-FAM-added derivative, and a biotin-added derivative.

### (Nucleic acids encoding cell surface molecule specifically recognizing human mesothelin, IL-7, CCL19, etc.)

Examples of the nucleic acid encoding a cell surface molecule specifically recognizing human mesothelin, the nucleic acid encoding IL-7, and the nucleic acid encoding CCL19 can include respective nucleic acids derived from a mammal and can preferably include respective nucleic acids derived from a human. Each of the nucleic acids can be appropriately selected according to the type of the cell to which the expression vector of the present invention is to be introduced. Sequence information on each of the nucleic acids can be appropriately obtained by the search of a publicly known document or a database such as NCBI (http://www.ncbi.nlm.nih.gov/guide/).

Examples of the nucleic acid encoding a cell surface molecule specifically recognizing human mesothelin can include a nucleic acid encoding CAR specifically recognizing human mesothelin.

The nucleic acid encoding single chain antibody comprised in the CAR specifically recognizing human mesothelin includes the following nucleic acid (1-1D)
(1-1D) a nucleic acid encoding single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18.

A specific example of the nucleic acid encoding single chain antibody comprised in the CAR specifically recognizing human mesothelin is the following nucleic acid (1-3D):
(1-3D) a nucleic acid encoding single chain antibody comprising a heavy chain variable region consisting of the amino acid sequence shown by SEQ ID NO: 1, and a light chain variable region consisting of the amino acid sequence shown by SEQ ID NO: 2.

Examples of the nucleic acid encoding a polypeptide of a transmembrane region comprised in the CAR can include a nucleic acid encoding a polypeptide of a human CD8 transmembrane region comprising an amino acid sequence having 85% or higher, preferably 90% or higher, more preferably 95% or higher, further preferably 98% or higher sequence identity to the amino acid sequence shown by SEQ ID NO: 7. Examples of the nucleic acids encoding polypeptides of CD28, 4-1BB, and CD3ζ intracellular regions in an immunocompetent cell activating signaling region comprised in the CAR can include a nucleic acid encoding a polypeptide that comprises an amino acid sequence having 85% or higher, preferably 90% or higher, more preferably 95% or higher, further preferably 98% or higher sequence identity to the amino acid sequence of the human CD28 intracellular region shown by SEQ ID NO: 8, and has action equivalent to that of the amino acid sequence shown by SEQ ID NO: 8, a nucleic acid encoding a polypeptide that comprises an amino acid sequence having 85% or higher, preferably 90% or higher, more preferably 95% or higher, further preferably 98% or higher sequence identity to the amino acid sequence of the human 4-1BB intracellular region shown by SEQ ID NO: 9, and has action equivalent to that of the amino acid sequence shown by SEQ ID NO: 9, and a nucleic acid encoding a polypeptide that comprises an amino acid sequence having 85% or higher, preferably 90% or higher, more preferably 95% or higher, further preferably 98% or higher sequence identity to the amino acid sequence of the human CD3ζ intracellular region shown by SEQ ID NO: 10, and has action equivalent to that of the amino acid sequence shown by SEQ ID NO: 10, and a combination thereof, preferably a nucleic acid encoding a polypeptide of the human CD28 intracellular region shown in SEQ ID NO: 8, a nucleic acid encoding a polypeptide of the human 4-1BB intracellular region shown in SEQ ID NO: 9, and a nucleic acid encoding a polypeptide of the human CD3ζ intracellular region shown in SEQ ID NO: 10 in order from the upstream side (5'-terminal side).

Examples of the nucleic acid encoding IL-7 can include a nucleic acid encoding a polypeptide that comprises an amino acid sequence having 85% or higher, preferably 90% or higher, more preferably 95% or higher, further preferably 98% or higher sequence identity to the amino acid sequence shown by SEQ ID NO: 28, and has action equivalent to that of the amino acid sequence shown by SEQ ID NO: 28, specifically, a nucleic acid consisting of the nucleotide sequence shown by SEQ ID NO: 30. The nucleic acid encoding IL-7 may be a nucleic acid having 80% or higher, preferably 85% or higher, more preferably 90% or higher, further preferably 95% or higher, most preferably 98% or higher sequence identity to the nucleic acid consisting of the nucleotide sequence shown by SEQ ID NO: 30 as long as the nucleic acid has action of enhancing a cell proliferation rate or a cell survival rate by IL-7. Examples of the nucleic acid encoding CCL19 can include a nucleic acid encoding a polypeptide that comprises an amino acid sequence having 85% or higher, preferably 90% or higher, more preferably 95% or higher, further preferably 98% or higher sequence identity to the amino acid sequence shown by SEQ ID NO: 29, and has action equivalent to that of the amino acid sequence shown by SEQ ID NO: 29, specifically, a nucleic acid consisting of the nucleotide sequence shown by SEQ ID NO: 31. A nucleic acid having 80% or higher, preferably 85% or higher, more preferably 90% or higher, further preferably 95% or higher, most preferably 98% or higher sequence identity to the nucleic acid consisting of the nucleotide sequence shown by SEQ ID NO: 31 may be used as long as the nucleic acid has the cell migrating action of CCL19.

### (Suicide gene)

The expression vector of the present invention may comprise a nucleic acid encoding a suicide gene. The suicide gene means a gene that directly or secondarily induces a substance having cellular toxicity through its expression and has the function of killing its own cell. Owing to the expression vector of the present invention comprising the nucleic acid encoding the suicide gene, for example, an immunocompetent cell present in a living body can be controlled by the administration of a drug activating the function of the suicide gene after disappearance of tumor according to the course of treatment of cancer. IL-7 or CCL19, unlike other cytokines, is less likely to cause cytokine release syndrome or tumorigenic transformation of transfected cells as adverse reactions. However, the enhanced functions of the immunocompetent cell harboring the expression vector of the present invention may cause unexpected influence of cytokines, etc. released upon attack on a target cancer tissue on neighboring tissues. In such a case, the nucleic acid encoding the suicide gene, comprised in the expression vector of the present invention is capable of reliably reducing a risk of cytokine release syndrome.

Examples of the suicide gene can include a gene encoding herpes simplex virus thymidine kinase (HSV-TK) or inducible caspase 9 described in a document given below. Examples of the drug activating the function of such a gene can include ganciclovir for the former and AP1903 which is chemical induction of dimerization (CID) for the latter.

### (Obtainment of nucleic acid sequence information and production of nucleic acid)

Each of the nucleic acids comprised in the expression vector of the present invention may be a naturally derived nucleic acid or an artificially synthesized nucleic acid, and can be appropriately selected according to the type of the cell to which the expression vector of the present invention is to be introduced. Their sequence information can be appropriately obtained by the search of a publicly known document or a database such as NCBI (http://www.ncbi.nlm.nih.gov/guide/).

Each of the nucleic acids can be produced by a publicly known technique such as a chemical synthesis method or a PCR amplification method on the basis of information on the nucleotide sequence of each of the nucleic acids. Codons selected for encoding amino acids may be engineered in order to optimize nucleic acid expression in host cells of interest.

### (Arrangement of each nucleic acid)

Any of the nucleic acids of the expression vector of the present invention may be arranged upstream or downstream of any of the nucleic acids. Specifically, the arrangement may be the nucleic acid encoding anti-human mesothelin CAR, the nucleic acid encoding IL-7 and the nucleic acid encoding CCL19, may be the nucleic acid encoding anti-human mesothelin CAR, the nucleic acid encoding CCL19 and the nucleic acid encoding IL-7, may be the nucleic acid encoding IL-7, the nucleic acid encoding CCL19 and the nucleic acid encoding anti-human mesothelin CAR, may be the nucleic acid encoding IL-7, the nucleic acid encoding anti-mesothelin CAR and the nucleic acid encoding CCL19, may be the nucleic acid encoding CCL19, the nucleic acid encoding anti-mesothelin CAR, and the nucleic acid encoding IL-7, or may be the nucleic acid encoding CCL19, the nucleic acid encoding IL-7 and the nucleic acid encoding anti-human mesothelin CAR, in order from the upstream side (5'-terminal side).

### (Transcription)

The nucleic acid encoding a CAR specifically recognizing mesothelin, the nucleic acid encoding IL-7, the nucleic acid encoding CCL19, and the nucleic acid encoding a suicide gene may be transcribed under different promoters or may be transcribed under one promoter using an internal ribosome entry site (IRES) or self-cleaving 2A peptide.

An arbitrary nucleic acid may be comprised between the nucleic acid encoding IL-7 and the nucleic acid encoding CCL19 in the case of transcribing these nucleic acids under one promoter using an internal ribosome entry site (IRES) or self-cleaving 2A peptide, or between the nucleic acid encoding a CAR specifically recognizing mesothelin and the nucleic acid encoding IL-7 and the nucleic acid encoding CCL19 in the case of comprising the nucleic acid encoding a CAR specifically recognizing mesothelin, as long as each of the nucleic acids can be expressed. The linking is preferably achieved via a sequence encoding self-cleaving peptide (2A peptide) or IRES, preferably a sequence encoding 2A peptide. The linking using such a sequence permits efficient expression of each of the nucleic acids.

The 2A peptide is a virus-derived self-cleaving peptide and is characterized in that the amino acid sequence shown by SEQ ID NO: 32 is cleaved at G-P (position of one residue from the C terminus) in the endoplasmic reticulum (Szymczak et al., Expert Opin. Biol. Ther. 5 (5): 627-638 (2005)). Hence, nucleic acids flanking the 2A peptide are each expressed independently in a cell via the 2A peptide.

The 2A peptide is preferably 2A peptide derived from picornavirus, rotavirus, insect virus, Aphthovirus or Trypanosoma virus, more preferably picornavirus-derived 2A peptide (F2A) shown in SEQ ID NO: 33.

### (Type of vector)

The type of the vector for the expression vector of the present invention may be a linear form or a circular form and may be a non-viral vector such as a plasmid, may be a viral vector, or may be a vector based on a transposon. Such a vector may contain a control sequence such as a promoter or a terminator, or a selective marker sequence such as a drug resistance gene or a reporter gene. The nucleic acid encoding IL-7 and the nucleic acid encoding CCL19 are operably arranged downstream of the promoter sequence so that each of the nucleic acids can be efficiently transcribed.

Examples of the promoter can include: a virus-derived promoter such as retrovirus LTR promoter, SV40 early promoter, cytomegalovirus promoter, and herpes simplex virus thymidine kinase promoter; and a mammal-derived promoter such as phosphoglycerate kinase (PGK) promoter, Xist promoter, β-actin promoter, and RNA polymerase II promoter. Alternatively, tetracycline-responsive promoter which is induced by tetracycline, Mx1 promoter which is induced by interferon, or the like may be used. Use of the promoter which is induced by a particular substance in the expression vector of the present invention permits control of induction of IL-7 and CCL19 expression according to the course of treatment of cancer, for example, when the immunocompetent cell containing the vector of the present invention is used as a pharmaceutical composition for use in the treatment of cancer.

Examples of the viral vector can include a retrovirus vector, a lentivirus vector, an adenovirus vector, and an adeno-associated virus vector and can preferably include a retrovirus vector, more preferably a pMSGV vector (Tamada k et al., Clin Cancer Res 18: 6436-6445 (2002)) and a pMSCV vector (manufactured by Takara Bio Inc.). Use of a retrovirus vector permits long-term and stable expression of a transgene because the transgene is integrated in the genome of a host cell.

In order to confirm the containment of the expression vector of the present invention in the immunocompetent cell, for example, the expression of CAR can be examined by flow cytometry, Northern blotting, Southern blotting, PCR such as RT-PCR, ELISA, or Western blotting when the expression vector contains a nucleic acid encoding CAR, and the expression of a marker gene inserted in the expression vector of the present invention can be examined when the expression vector contains the marker gene.

### (Pharmaceutical composition)

The pharmaceutical composition of the present invention is not limited as long as the pharmaceutical composition comprises the immunocompetent cell of the present invention and a pharmaceutically acceptable additive. Examples of the additive can include saline, buffered saline, a cell culture medium, dextrose, injectable water, glycerol, ethanol, and a combination thereof, a stabilizer, a solubilizer and a surfactant, a buffer and an antiseptic, a tonicity agent, a filler, and a lubricant. Since the immunocompetent cell in the pharmaceutical composition of the present invention has a signaling region that induces the activation of the immunocompetent cell, the pharmaceutical composition of the present invention may serve as a pharmaceutical composition for use in the treatment of cancer. Such a pharmaceutical composition for use in the treatment of cancer may contain a package insert, a label, a package, or the like stating a use method, etc. for use in the treatment of cancer. Since the immunocompetent cell in the pharmaceutical composition of the present invention has suppressive effects on tumor recurrence, the pharmaceutical composition of the present invention may serve as a pharmaceutical composition for use in the suppression of tumor recurrence. Such a pharmaceutical composition for use in the suppression of tumor recurrence may contain a package insert, a label, a package, or the like stating a use method, etc. for use in the suppression of tumor recurrence.

The pharmaceutical composition of the present invention can be administered to a test subject in need thereof by use of a method known to those skilled in the art. Examples of the administration method can include intravenous, intratumoral, intracutaneous, subcutaneous, intramuscular, intraperitoneal, intraarterial, intramedullary, intracardiac, intraarticular, intrasynovial, intracranial, intrathecal, and subarachnoidal (spinal fluid) injection.

In an exemplary method, the pharmaceutical composition of the present invention can be independently administered in one portion or several divided portions 4 times, 3 times, twice, or once a day, at a 1-day, 2-day, 3-day, 4-day, or 5-day interval, once a week, at a 7-day, 8-day, or 9-day interval, twice a week, once a month, or twice a month.

The cancer for the pharmaceutical composition of the present invention is not particularly limited and is preferably a cancer type expressing mesothelin in a cancer tissue, or a cancer type derived from cancer cells expressing mesothelin. Examples thereof can include: cancer such as mesothelioma, colorectal cancer (colon cancer or rectum cancer), pancreatic cancer, thymic cancer, bile duct cancer, lung cancer (adenocarcinoma, squamous cell cancer, adenosquamous cancer, undifferentiated cancer, large-cell cancer, and small-cell cancer), skin cancer, breast cancer, prostate cancer, urinary bladder cancer, vaginal cancer, neck cancer, uterine cancer, liver cancer, kidney cancer, pancreatic cancer, spleen cancer, tracheal cancer, bronchial cancer, colon cancer, small intestine cancer, stomach cancer, esophageal cancer, gallbladder cancer, testis cancer, and ovary cancer; cancer of a bone tissue, a cartilage tissue, a fat tissue, a muscle tissue, a vascular tissue, and a hematopoietic tissue; sarcoma such as chondrosarcoma, Ewing's sarcoma, malignant hemangioendothelioma, malignant schwannoma, osteosarcoma, and soft tissue sarcoma; blastoma such as hepatoblastoma, medulloblastoma, nephroblastoma, neuroblastoma, pancreatoblastoma, pleuropulmonary blastoma, and retinoblastoma; and embryonic cell tumor. The dose of the pharmaceutical composition to be administered can be a therapeutically effective amount. Examples thereof can preferably include 1 × 10⁴ to 1 × 10¹⁰ cells, preferably 1 × 10⁵ to 1 × 10⁹ cells, more preferably 5 × 10⁶ to 5 × 10⁸ cells, in terms of the number of cells to be administered in a single dose.

The pharmaceutical composition of the present invention can be used in combination with an additional anticancer agent. Examples of the additional anticancer agent can include: an alkylating agent such as cyclophosphamide, bendamustine, ifosfamide, and dacarbazine; an antimetabolite such as pentostatin, fludarabine, cladribine, methotrexate, 5-fluorouracil, 6-mercaptopurine, and enocitabine; a molecular targeting drug such as rituximab, cetuximab, and trastuzumab; a kinase inhibitor such as imatinib, gefitinib, erlotinib, afatinib, dasatinib, sunitinib, and trametinib; a proteasome inhibitor such as bortezomib; a calcineurin inhibitory drug such as cyclosporine and tacrolimus; an anticancer antibiotic such as idarubicin, doxorubicin, and mitomycin C; a vegetable alkaloid such as irinotecan and etoposide; a platinum-containing drug such as cisplatin, oxaliplatin, and carboplatin; a hormone therapeutic such as tamoxifen and bicalutamide; and an immunoregulatory drug such as interferon, nivolumab, and pembrolizumab.

Examples of the method for "using the pharmaceutical composition of the present invention in combination with the additional anticancer agent" can include a method of using the additional anticancer agent in a process and then using the pharmaceutical composition of the present invention, a method of concurrently using the pharmaceutical composition of the present invention and the additional anticancer agent, and a method of using the pharmaceutical composition of the present invention in a process and then using the additional anticancer agent. Combined use of the pharmaceutical composition of the present invention for use in the treatment of cancer with the additional anticancer agent further improves therapeutic effects on cancer and can reduce the adverse reactions of each anticancer agent by decreasing the administration frequency or dose of the anticancer agent. Alternatively, the additional anticancer agent may be contained in the pharmaceutical composition of the present invention.

Hereinafter, the present invention will be described more specifically with reference to Examples. However, the technical scope of the present invention is not limited by these examples.

### [Example 1] Production of anti-human mesothelin CAR (Synthesis of scFv sequence and DNA fragment of anti-human mesothelin CAR)

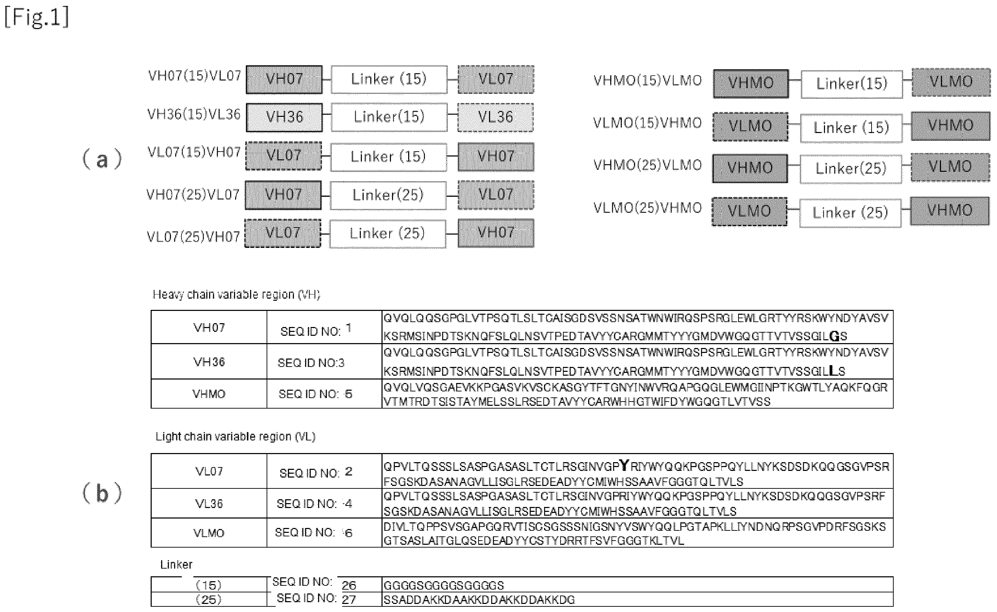

The sequences of 9 types of anti-human mesothelin scFvs shown in Figure 1 were designed in order to compare thereamong the sequences and order of VL and VH, and the type of a suitable signal peptide.

VH07(15)VL07 consists of the amino acid sequence of a heavy chain variable region shown by SEQ ID NO: 1, the amino acid sequence of a peptide linker shown by SEQ ID NO: 26, and the amino acid sequence of a light chain variable region shown by SEQ ID NO: 2.

VH36(15)VL36 consists of the amino acid sequence of a heavy chain variable region shown by SEQ ID NO: 3, the amino acid sequence of a peptide linker shown by SEQ ID NO: 26, and the amino acid sequence of a light chain variable region shown by SEQ ID NO: 4.

VL07(15)VH07 consists of the amino acid sequence of a light chain variable region shown by SEQ ID NO: 2, the amino acid sequence of a peptide linker shown by SEQ ID NO: 26, and the amino acid sequence of a heavy chain variable region shown by SEQ ID NO: 1.

VH07(25)VL07 consists of the amino acid sequence of a heavy chain variable region shown by SEQ ID NO: 1, the amino acid sequence of a peptide linker shown by SEQ ID NO: 27, and the amino acid sequence of a light chain variable region shown by SEQ ID NO: 2.

VL07(25)VH07 consists of the amino acid sequence of a light chain variable region shown by SEQ ID NO: 2, the amino acid sequence of a peptide linker shown by SEQ ID NO: 27, and the amino acid sequence of a heavy chain variable region shown by SEQ ID NO: 1.

VHMO(15)VLMO consists of the amino acid sequence of a heavy chain variable region shown by SEQ ID NO: 5, the amino acid sequence of a peptide linker shown by SEQ ID NO: 26, and the amino acid sequence of a light chain variable region shown by SEQ ID NO: 6.

VLMO(15)VHMO consists of the amino acid sequence of a light chain variable region shown by SEQ ID NO: 6, the amino acid sequence of a peptide linker shown by SEQ ID NO: 26, and the amino acid sequence of a heavy chain variable region shown by SEQ ID NO: 5.

VHMO(25)VLMO consists of the amino acid sequence of a heavy chain variable region shown by SEQ ID NO: 5, the amino acid sequence of a peptide linker shown by SEQ ID NO: 27, and the amino acid sequence of a light chain variable region shown by SEQ ID NO: 6.

VLMO(25)VHMO consists of the amino acid sequence of a light chain variable region shown by SEQ ID NO: 6, the amino acid sequence of a peptide linker shown by SEQ ID NO: 27, and the amino acid sequence of a heavy chain variable region shown by SEQ ID NO: 5.

The amino acid sequences shown by SEQ ID NO: 1 and SEQ ID NO: 3 differ in that the 127th amino acid is glycine (G) in SEQ ID NO: 1 and is leucine (L) in the amino acid sequence shown by SEQ ID NO: 3. The amino acid sequences shown by SEQ ID NO: 2 and SEQ ID NO: 4 differ in that the 33rd amino acid in SEQ ID NO: 2 is tyrosine (Y), which is deleted in the amino acid sequence shown by SEQ ID NO: 4.

A DNA fragment encoding the amino acid sequence of each of the anti-human mesothelin scFvs was synthesized.

### (Production of anti-human mesothelin IL-7/CCL19 CAR expression vector for expression of IL-7/CCL19 and HSV-TK, and Conv. anti-human mesothelin CAR expression vector without expression of IL-7/CCL19)

CAR-T cell therapy may cause systemic adverse reactions such as cytokine release syndrome due to strong immune response to a target antigen. A CAR construct harboring a herpes virus-derived thymidine kinase HSV-TK gene as a suicide gene was produced in order to cope with the problem. If T cells are transfected with the construct so that the CAR-expressing T cells express HSV-TK, the addition of a cytomegalovirus therapeutic drug ganciclovir induces the apoptosis of the CAR-T cells and kills these cells. Therefore, the CAR-T cells in the body are controllable by the administration of ganciclovir.

First, a third-generation CAR construct sequentially having anti-human mesothelin scFv, human CD8 transmembrane region and human CD28-4-1BB-CD3ζ intracellular signaling region from the N-terminal side was produced in accordance with the method described in Patent Document 2. 2A peptide F2A was added to the C terminus of the construct, and human IL-7-F2A-human CCL19-F2A-HSV-TK was further added to downstream thereof. The obtained construct sequentially having scFv, human CD8 transmembrane region, human CD28-4-1BB-CD3ζ intracellular signaling region, human IL-7, human CCL19, and HSV-TK was inserted to a pMSGV1 retrovirus expression vector (Tamada k et al., Clin Cancer Res 18: 6436-6445 (2012)) to produce a pMSGV vector for expression of anti-human mesothelin scFv, human CD8 transmembrane region, human CD28-4-1BB-CD3ζ intracellular signaling region, human IL-7, human CCL19, and HSV-TK. Next, the anti-human mesothelin scFv region in the pMSGV vector was replaced by restriction enzyme (NcoI and NotI) treatment and ligation with each anti-human mesothelin scFv DNA fragment synthesized by the method described in the section "Synthesis of scFv sequence and DNA fragment of anti-human mesothelin CAR" to produce each "IL-7/CCL19 expression-anti-human mesothelin CAR vector". The pMSGV1 vector has immune globulin G-derived signal peptide T (SEQ ID NO: 11) on the N-terminal side of scFv. As for the vector having the VH07(15)VL07 DNA fragment replaced for the scFv region, the signal peptide T shown in SEQ ID NO: 11 was replaced with signal peptide P shown in SEQ ID NO: 12 as a signal peptide for production. In addition, a "Conv. anti-human mesothelin CAR vector" was produced as a control without IL-7 and CCL19 in the same way as the method described above except that HSV-TK was used instead of the human IL-7-F2A-human CCL19-F2A-HSV-TK.

### (Production of retrovirus harboring IL-7/CCL19 expression-anti-human mesothelin CAR vector or Conv. anti-human mesothelin CAR vector)

Retrovirus was produced for transfection of T cells. A GP2-293 packaging cell line (manufactured by Takara Bio Inc.) was transfected with each of the IL-7/CCL19 expression-anti-human mesothelin CAR vectors or the Conv. anti-human mesothelin CAR vector and a p-Ampho plasmid (manufactured by Takara Bio Inc.) using Lipofectamine 3000 (manufactured by Life Technologies Corp.) to produce retrovirus harboring the IL-7/CCL19 expression-anti-human mesothelin CAR vector or the Conv. anti-human mesothelin CAR vector. A supernatant containing the retrovirus was recovered 48 hours after the transfection.

The culture solution used for the GP2-293 cells was DMEM supplemented with 10% FCS and 1% penicillin-streptomycin (manufactured by Wako Pure Chemical Industries, Ltd.). The culture solution used for T cells for use in Examples mentioned later was GT-T551 containing 2.0% human AB blood type serum (manufactured by Sigma-Aldrich Co. LLC), 1% penicillin-streptomycin (manufactured by Wako Pure Chemical Industries, Ltd.), and 2.5 µg/ml amphotericin B (manufactured by Bristol-Myers Squibb Company).

### (Transduction of T cell)

2 × 10⁶ peripheral blood mononuclear cells collected from the blood of a healthy donor were cultured with IL-2 (manufactured by PeproTech, Inc.) at 37°C for 3 days in a 5% CO₂ incubator on a plate on which an anti-CD3 monoclonal antibody (5 µg/ml) and RetroNectin(R) (manufactured by Takara Bio Inc., 25 µg/ml) were immobilized for activation of T cells. On day 2 after the start of culture, the supernatant containing the produced retrovirus harboring the IL-7/CCL19 expression-anti-human mesothelin CAR vector or the Conv. anti-human mesothelin CAR vector was added at 500 µl/well to a surface-untreated 24-well plate coated in advance with 25 µg/ml RetroNectin (manufactured by Takara Bio Inc.), and centrifuged at 2000 g for 2 hours to produce a retrovirus preload plate. A total of two such plates were produced, washed with 1.5% BSA/PBS after the completion of centrifugation, and stored at 4°C until use. On culture day 3, the activated cells were recovered from the plate and adjusted as a cell suspension (1 × 10⁵ cells/ml). This cell suspension was added at 1 ml/well to the retrovirus preload plate and cultured at 37°C for 24 hours in the presence of IL-2 in a 5% CO₂ incubator for the first retrovirus infection. On the next day (culture day 4), the cell lysate in each well was transferred to the stored second virus preload plate, centrifuged at 500 g for 1 minute, and then cultured at 37°C for 4 hours for the second infection. After the culture at 37°C for 4 hours, 1 ml of the cell suspension in each well was transferred to a fresh 12-well cell culture plate, diluted 4-fold with a fresh culture solution (GT-T551) containing IL-2, and cultured at 37°C in a 5% CO₂ incubator. The culture was continued up to 7 days counted from the start date of culture of the peripheral blood mononuclear cells to obtain "anti-human mesothelin CAR-IL-7/CCL19-expressing T cells" as T cells harboring the IL-7/CCL19 expression-anti-human mesothelin CAR vector or "anti-human mesothelin CAR-expressing T cells" as T cells harboring the Conv. anti-human mesothelin CAR vector. The anti-human mesothelin CAR-IL-7/CCL19-expressing T cells contained an exogenous nucleic acid encoding anti-human mesothelin CAR, an exogenous nucleic acid encoding IL-7, and an exogenous nucleic acid encoding CCL19. The anti-human mesothelin CAR-expressing T cells contained a nucleic acid encoding anti-human mesothelin CAR and contained neither an exogenous nucleic acid encoding IL-7 nor an exogenous nucleic acid encoding CCL19. At the same time therewith, "CAR, IL-7, and CCL19 non-expressing T cells" (non-transfected cells: Non-infection) were produced as a CAR-negative cell control by activating peripheral blood mononuclear cells obtained from the same healthy donor by the same approach as above, but not infecting the cells with the retrovirus.

Here, the retrovirus vector was used, as described above, for introducing the nucleic acid encoding anti-human mesothelin CAR, the nucleic acid encoding IL-7, and the nucleic acid encoding CCL19 to T cells. Hence, when T cells harboring these nucleic acids proliferate by culture, some of the T cells contain the retrovirus vector in their cytoplasms. However, in most of these T cells, the nucleic acid encoding anti-human mesothelin CAR, the nucleic acid encoding IL-7, and the nucleic acid encoding CCL19 are integrated in the genome. When the nucleic acid encoding anti-human mesothelin CAR, the nucleic acid encoding IL-7, and the nucleic acid encoding CCL19 are integrated in the genome of these T cells, anti-human mesothelin CAR, IL-7, and CCL19 are expressed from the exogenous recombinant construct introduced therein.

### [Example 2] Measurement of CAR expression by flow cytometry

### (Flow cytometry analysis)

The expression level of CAR recognizing mesothelin as an antigen was analyzed by flow cytometry analysis. The produced anti-human mesothelin CAR-IL-7/CCL19-expressing T cells were stained through reaction with recombinant human mesothelin (C-terminally containing 6-His) (manufactured by BioLegend, Inc.), phycoerythrin (PE)-labeled anti-6-His monoclonal antibody (manufactured by Abcam plc), and allophycocyanin (APC)-labeled anti-CD8 monoclonal antibody (manufactured by Affymetrix/Thermo Fisher Scientific Inc.). The flow cytometer used was EC800 (manufactured by Sony Corp.). The data was analyzed using FlowJo software (manufactured by Tree Star, Inc.).

### (Results)

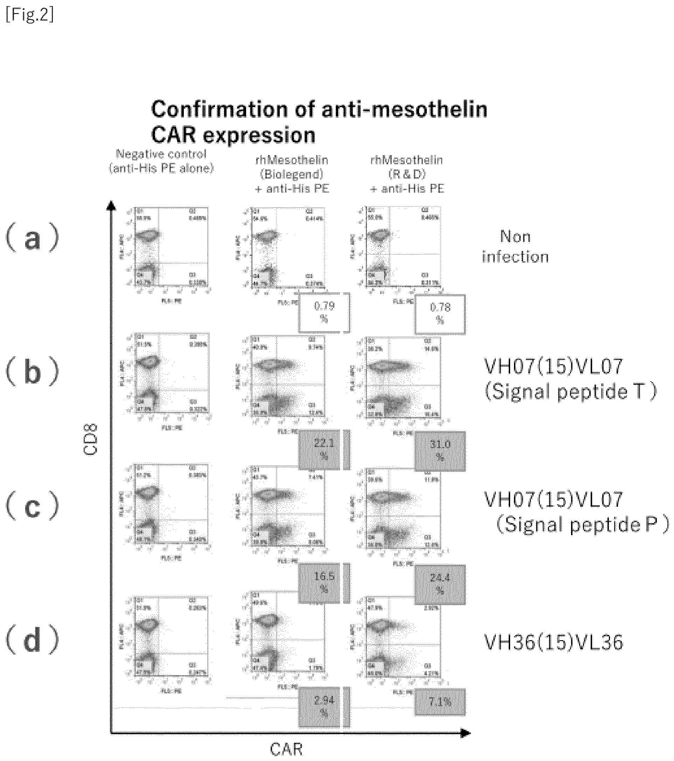

First, results of flow cytometry analysis on the anti-human mesothelin CAR-IL-7/CCL19-expressing T cells having VH07(15)VL07 (expressed through signal peptide T), VH07(15)VL07 (expressed through signal peptide P) or VH36(15)VL36 as the scFv region are shown in Figure 2. In Figure 2, the horizontal axis of each graph depicts the expression of CAR, and the longitudinal axis depicts the expression of CD8. As shown in Figure 2, all the three types of anti-human mesothelin CAR-IL-7/CCL19-expressing T cells were confirmed to highly express CAR as compared with the CAR, IL-7, and CCL19 non-expressing T cells (Non-infection).

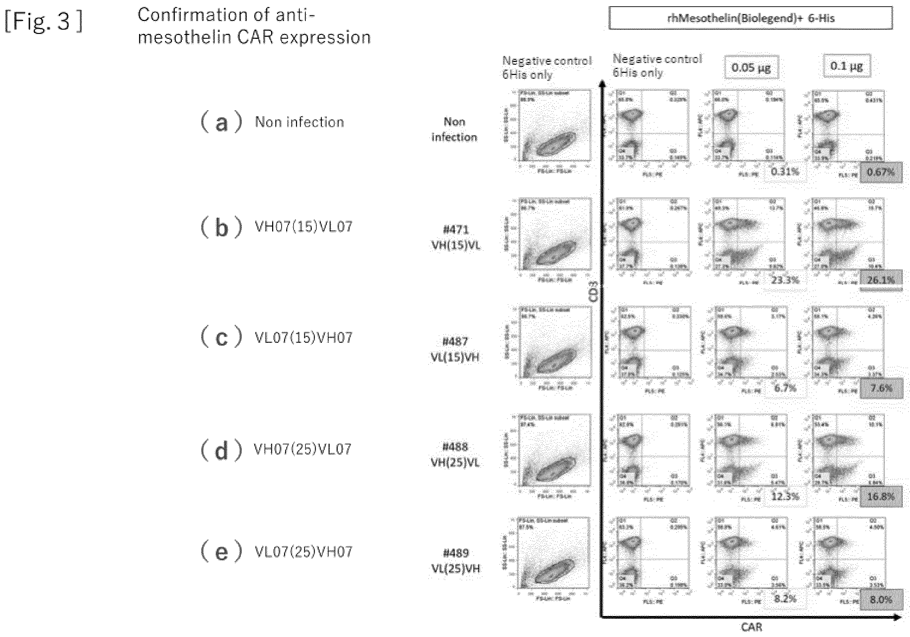

Next, results of flow cytometry analysis on the anti-human mesothelin CAR-IL-7/CCL19-expressing T cells having VH07(15)VL07, VL07(15)VH07, VH07(25)VL07, or VL07(25)VH07 as the scFv region are shown in Figure 3. In Figure 3, the abscissa of each graph depicts the expression of CAR, and the ordinate depicts the expression of CD8. Figure 3(a) shows results about the CAR, IL-7, and CCL19 non-expressing T cells (Non-infection), and Figures 3(b) to 3(e) show results about the anti-human mesothelin CAR-IL-7/CCL19-expressing T cells having each scFv region. The numerical values in the drawing represent the percentage of each population. As shown in Figures 3(b) to 3(e), the expression of CAR was confirmed in the anti-human mesothelin CAR-IL-7/CCL19-expressing T cells.

### [Example 3] Mesothelin expression of each tumor cell

### (Flow cytometry analysis)

The expression level of mesothelin in each tumor cell line was confirmed in order to confirm a tumor cell line expressing mesothelin. Malignant mesothelioma cell lines ACC-MESO-1, Y-MESO8A, NCI-H2052, NCI-H226, and MSTO211H, and a kidney cancer cell line A498 were stained with a commercially available anti-mesothelin antibody (Catalog Number FAB32652P: manufactured by R&D Systems, Inc.) labeled with PE. The expression of mesothelin in each tumor cell was measured by flow cytometry analysis. The staining with the PE-labeled anti-human mesothelin antibody was performed in 3 µg/sample. The flow cytometer used was EC800 (manufactured by Sony Corp.) . The data was analyzed using FlowJo software (manufactured by Tree Star, Inc.).

### (Results)

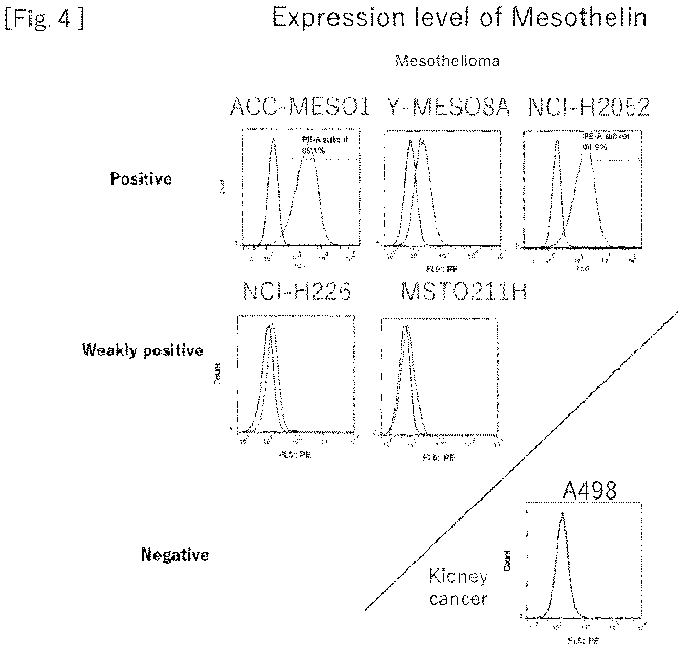

The results are shown in Figure 4. The expression of mesothelin was confirmed in the malignant mesothelioma cell lines ACC-MESO-1, Y-MESO8A, NCI-H2052, NCI-H226, and MSTO211H. On the other hand, the expression of mesothelin was not confirmed in the kidney cancer cell line A498.

### [Example 4] Cytotoxicity test - 1

### (Co-culture test)

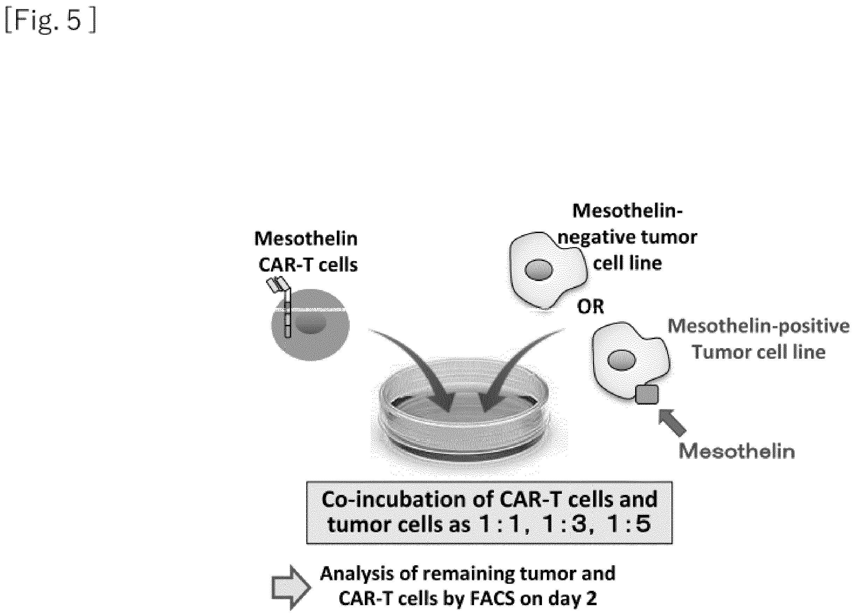

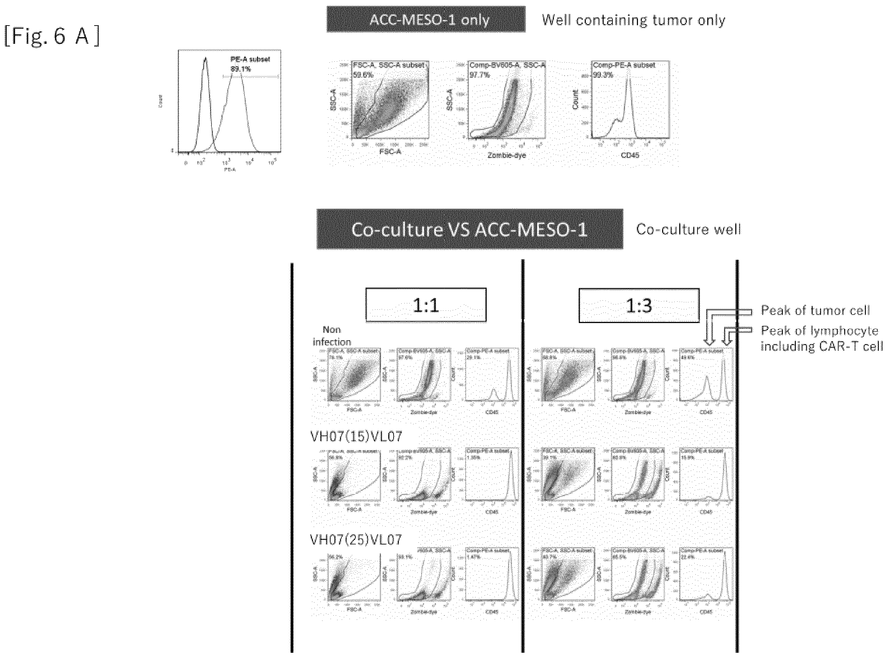

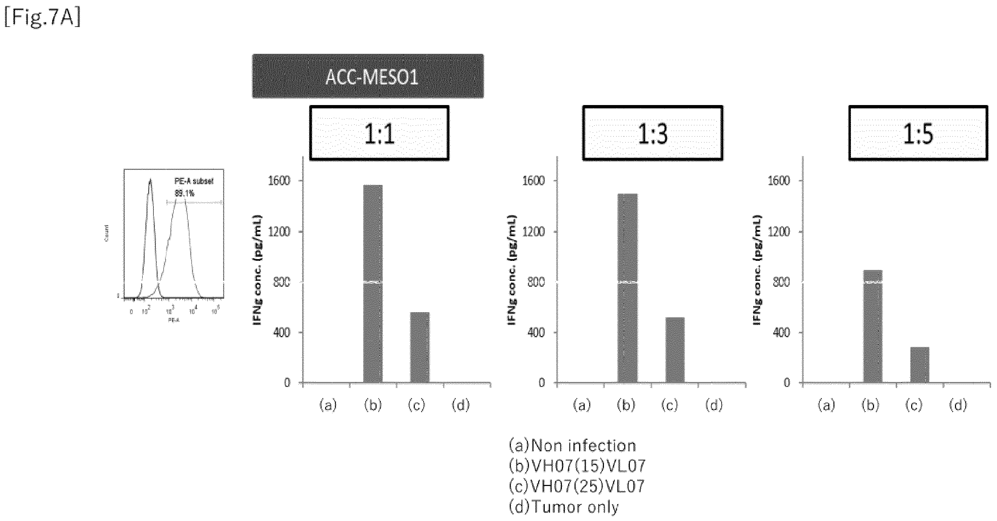

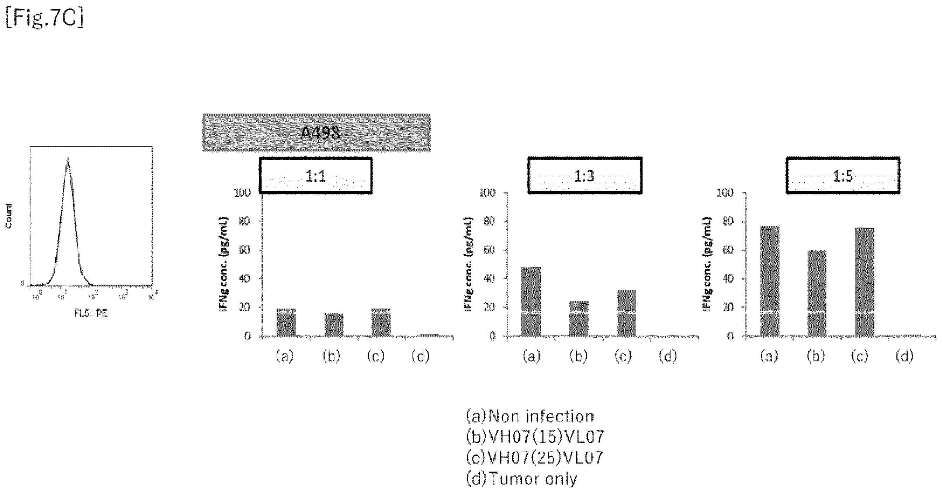

The anti-human mesothelin CAR-IL-7/CCL19-expressing T cells (scFv region: VH07(15)VL07 or VH07(25)VL07) used as an effector were adjusted to an effector:tumor cell ratio of 1:1, 1:3, or 1:5 (1:5: only for measurement and analysis of IFN-γ) with a mesothelin-positive tumor cell line (ACC-MESO-1 or NCI-H2052) or a mesothelin-negative tumor cell line (A498) on a culture plate and then co-cultured at 37°C in an incubator. This co-culture is illustrated in Figure 5. The culture solution used was RPMI containing 10% fetal calf serum (FCS), 1% penicillin-streptomycin (manufactured by Wako Pure Chemical Industries, Ltd.), 50 µM 2-ME (manufactured by Gibco/Thermo Fisher Scientific Inc.) and 25 mM HEPES (manufactured by Sigma-Aldrich Co. LLC). A tumor cell line surviving 2 days after the start of co-culture was measured by flow cytometry while IFN-γ produced into the culture supernatant was measured using a commercially available IFN-γ ELISA kit (manufactured by BioLegend, Inc.). The results of measuring a tumor cell line surviving 2 days after the start of co-culture by flow cytometry are shown in Figures 6A to 6C, and the results of measuring produced IFN-γ after the co-culture are shown in Figures 7A to 7C. The CAR, IL-7, and CCL19 non-expressing T cells (Non infection) were used as a control for the anti-human mesothelin CAR-IL-7/CCL19-expressing T cells. For the flow cytometry, dead cells were distinguished from live cells by staining with Zombie Yellow (manufactured by BioLegend, Inc.), and T cells were stained with PE-labeled anti-CD45 monoclonal antibody (manufactured by BioLegend, Inc.). The flow cytometer used was BD LSRFortessa X-20 (manufactured by BD Biosciences). The data was analyzed using FlowJo software (manufactured by Tree Star, Inc.).

### (Results)

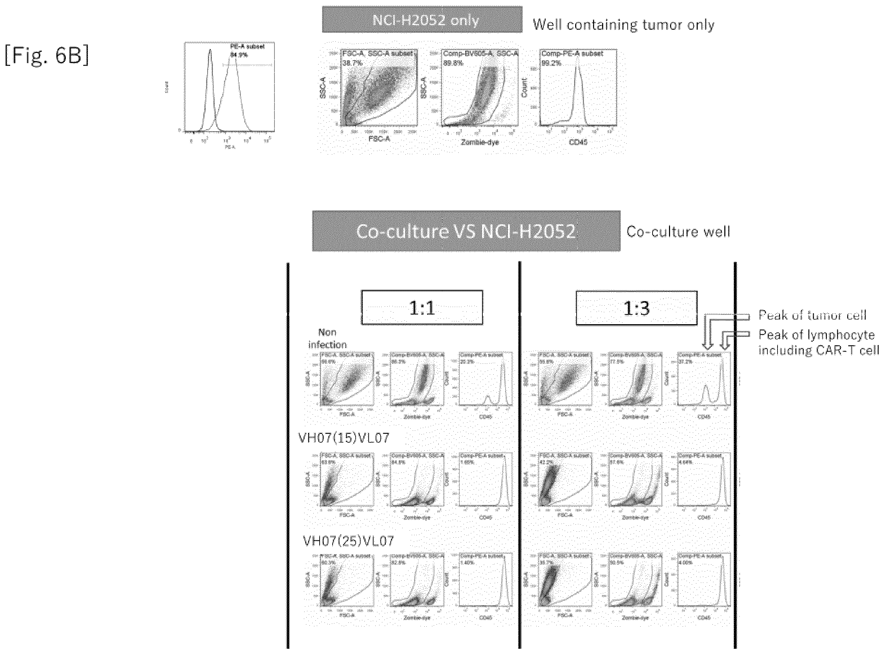

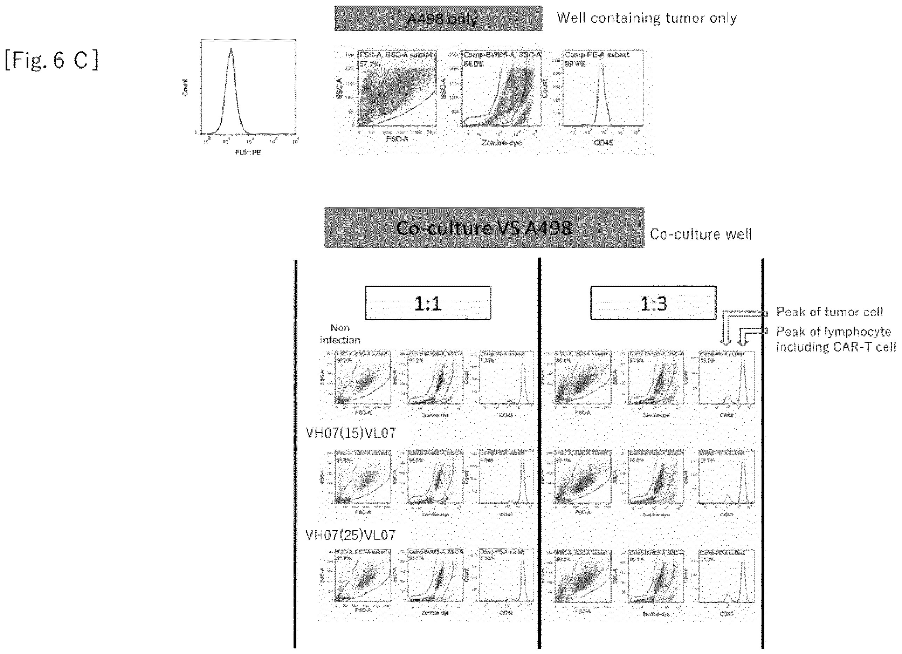

As shown in Figures 6A to 6C, all the target tumor cells were shown to proliferate at the same level as in wells containing tumor only, by the co-culture of the control CAR, IL-7, and CCL19 non-expressing T cells (Non-infection) with each target tumor cell. On the other hand, for the anti-human mesothelin CAR-IL-7/CCL19-expressing T cells, the target tumor cells proliferated, as with wells containing tumor only, by the co-culture with the mesothelin-negative target cells (A498: Figure 6C), whereas an evidently decreased number of tumor cells was observed in the co-culture with the mesothelin-positive tumor cells (ACC-MESO-1: Figure 6A, NCI-H2052: Figure 6B) as compared with wells containing the mesothelin-positive tumor cells only and wells of co-culture with the control cells. Thus, the anti-human mesothelin CAR-IL-7/CCL19-expressing T cells were able to be confirmed to damage tumor cells in an antigen-specific manner.

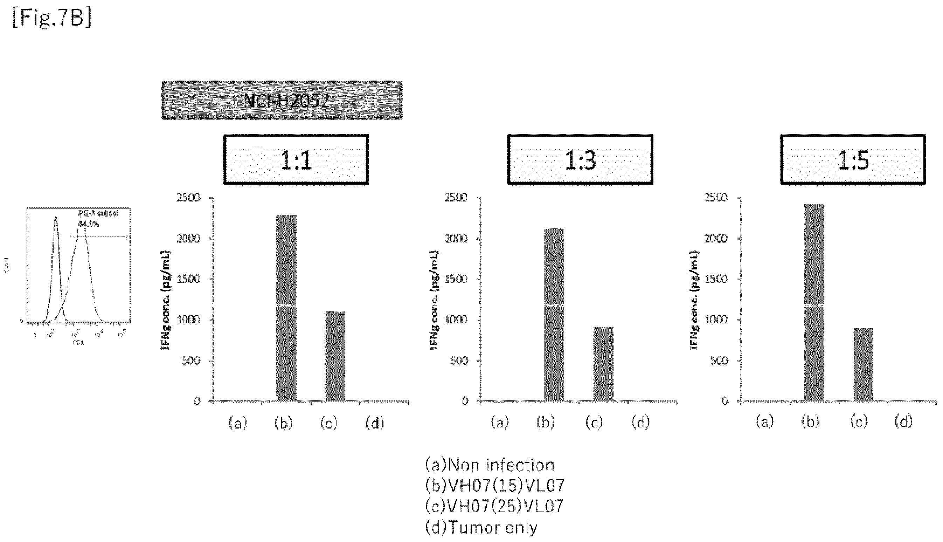

As shown in Figures 7A to 7C, the ELISA analysis of IFN-γ using the supernatant after the co-culture also confirmed marked production of IFN-γ only in the supernatant of the co-culture of the anti-human mesothelin CAR-IL-7/CCL19-expressing T cells with the mesothelin-positive tumor cells (ACC-MESO-1: Figure 7A, NCI-H2052: Figure 7B).

### [Example 5] Cytotoxicity test - 2

### (Co-culture test)

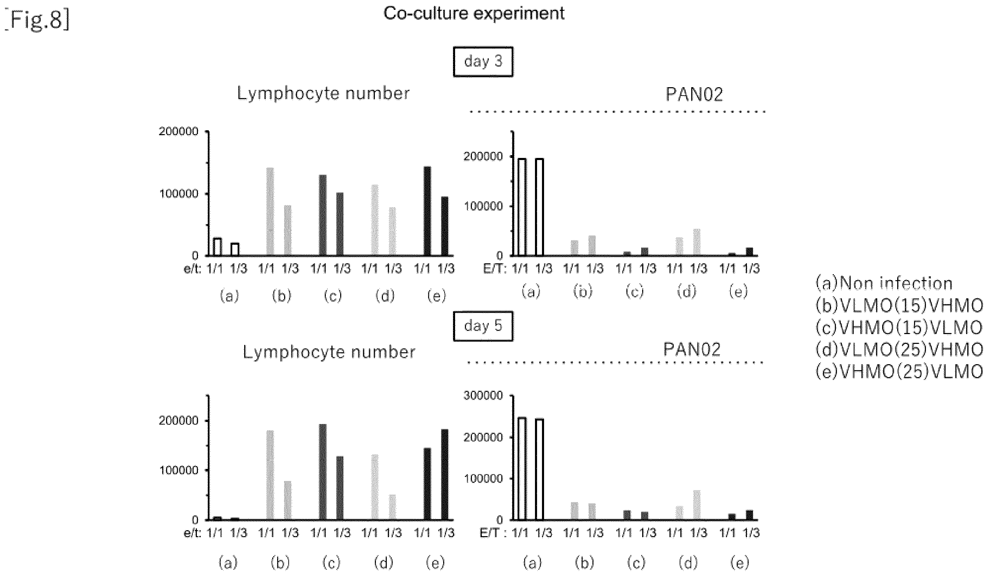

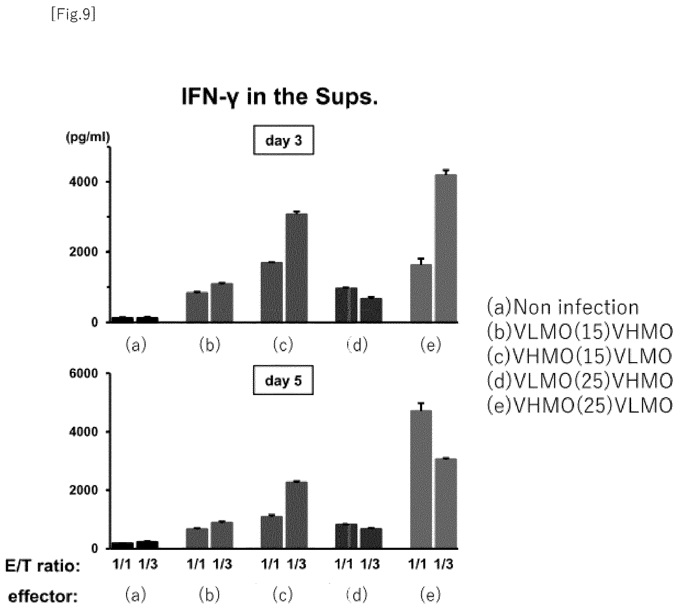

In the same way as the method of Example 4, the anti-human mesothelin CAR-IL-7/CCL19-expressing T cells (scFv region: VHMO(15)VLMO, VLMO(15)VHMO, VHMO(25)VLMO, or VLMO(25)VHMO) or the "CAR, IL-7, and CCL19 non-expressing T cells" (non-transfected cells: Non-infection) were adjusted to an effector:tumor cell ratio of 1:1 or 1:3 with a mesothelin-positive tumor cell line PAN02 or a mesothelin-negative tumor cell line on a culture plate and then co-cultured at 37°C in an incubator. Results of measuring leukocytes or a PAN02 tumor cell line surviving 3 days or 5 days after the start of co-culture by flow cytometry are shown in Figure 8, and results of measuring produced IFN-γ after the co-culture are shown in Figure 9. The T cells expressing anti-human mesothelin CAR, IL-7 and CCL19, used in this Example and "Therapeutic effect in tumor model" of Example 5 mentioned later were produced in accordance with the method of Example 1 (hereinafter, referred to as "anti-human mesothelin CAR-mouse IL-7/mouse CCL19-expressing mouse T cells") using, as the pMSGV1 retrovirus expression vector, a pMSGV1 retrovirus expression vector prepared so as to insert mouse IL-7-F2A-mouse CCL19-F2A-HSV-TK instead of the human IL-7-F2A-human CCL19-F2A-HSV-TK and mouse CD8 transmembrane region and mouse CD28-4-1BB-CD3ζ intracellular signaling region instead of the human CD8 transmembrane region and the human CD28-4-1BB-CD3ζ intracellular signaling region, and using spleen- and lymphocyte-derived mouse T cells as the T cells. The "CAR, IL-7, and CCL19 non-expressing T cells" (non-transfected cells: Non-infection) used in this Example were obtained using spleen- and lymphocyte-derived mouse T cells as the T cells.

### (Results)

As shown in Figure 8, the anti-human mesothelin CAR-mouse IL-7/mouse CCL19-expressing mouse T cells were able to be confirmed to damage tumor cells. As shown in Figure 9, the ELISA of IFN-γ using the supernatant after the co-culture also confirmed marked production of IFN-γ only in the supernatant of the co-culture of the anti-human mesothelin CAR-mouse IL-7/mouse CCL19-expressing mouse T cells with the PAN02 tumor cell line.

### [Example 6] Therapeutic effect in tumor model

### (Administration of anti-mouse mesothelin CAR-IL-7/CCL19-expressing T cell to tumor model mouse)

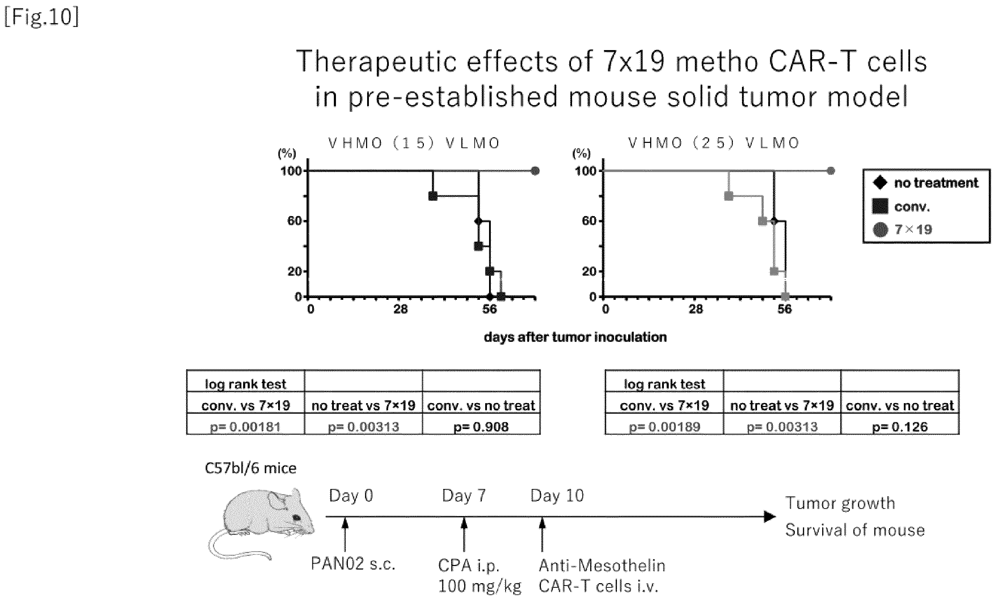

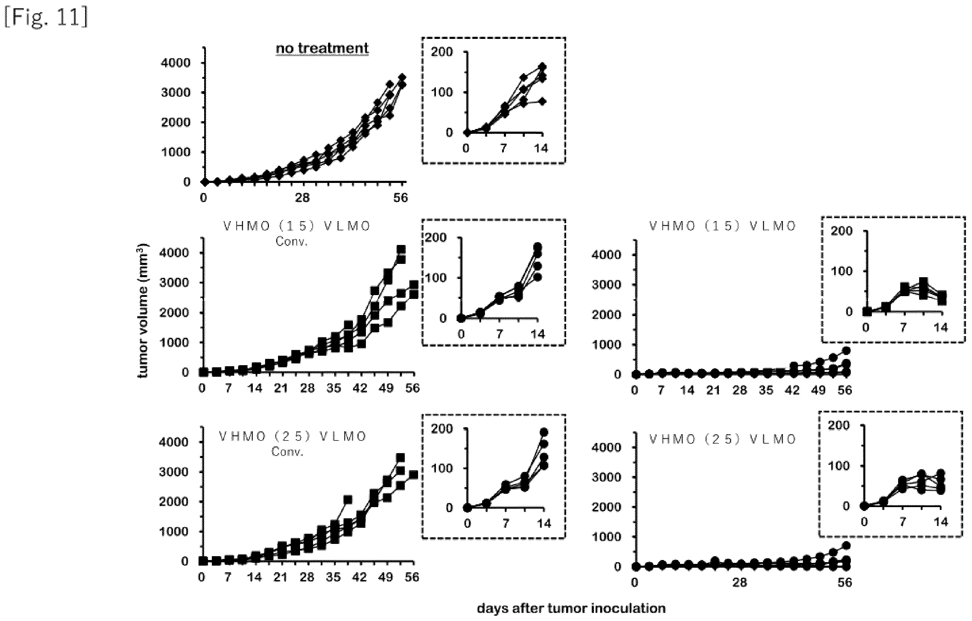

Seven- to ten-week-old C57BL/6 mice (purchased from Japan SLC, Inc.) were each subcutaneously inoculated with 5 × 10⁵ cells of a PAN02 pancreatic cancer cell line. On day 7 after the inoculation, an anticancer agent cyclophosphamide (CPA, 100 mg/kg) was intraperitoneally administered to the mice. On day 10, 1 × 10⁶ anti-human mesothelin CAR-mouse IL-7/mouse CCL19-expressing mouse T cells (scFv region: VHMO(15)VLMO, VLMO(15)VHMO, VHMO(25)VLMO, or VLMO(25)VHMO) or the "anti-human mesothelin CAR-expressing mouse T cells" produced in Example 5 were intravenously administered thereto. Results of mouse survival rates from VHMO(15)VLMO or VHMO(25)VLMO are shown in Figure 10, and results of tumor volumes from VHMO(15)VLMO, VLMO(15)VHMO, VHMO(25)VLMO, or VLMO(25)VHMO) are shown in Figure 11. In Figure 10, the abscissa depicts the number of days after the subcutaneous inoculation of PAN02 (the day on which PAN02 was subcutaneously inoculated to the mice was defined as day 0), and the ordinate depicts the survival rate. In Figure 11, the abscissa depicts the number of days after the subcutaneous inoculation of PAN02, and the ordinate depicts the tumor volume (major axis of the tumor × (minor axis of the tumor)² / 2 (mm³)). "no treatment" represents a group given CPA only, "Conv." represents a group given CPA and then the anti-human mesothelin CAR-expressing mouse T cells, and "7×19" represents a group given CPA and then the anti-human mesothelin CAR-mouse IL-7/mouse CCL19-expressing mouse T cells. The "anti-human mesothelin CAR-expressing mouse T cells" were produced in the same way as the method of Example 1 except that in the method for producing "anti-human mesothelin CAR-expressing T cells" described in Example 1, a pMSGV1 retrovirus expression vector prepared so as to insert HSV-TK instead of the human IL-7-F2A-human CCL19-F2A-HSV-TK and mouse CD8 transmembrane region and mouse CD28-4-1BB-CD3ζ intracellular signaling region instead of the human CD8 transmembrane region and the human CD28-4-1BB-CD3ζ intracellular signaling region was used as the pMSGV1 retrovirus expression vector, and spleen- and lymphocyte-derived mouse T cells were used as the T cells.

### (Results)

As shown in Figure 10, the administration of the anti-human mesothelin CAR-IL-7/CCL19-expressing T cells of the present invention was found to significantly elevate survival rates. As shown in Figure 11, the administration of the anti-human mesothelin CAR-IL-7/CCL19-expressing T cells of the present invention was found to evidently suppress tumor growth. This demonstrated that the anti-human mesothelin CAR-IL-7/CCL19-expressing T cells exhibit excellent antitumor activity in the tumor model mice.

### [Example 7] Therapeutic effect in tumor model - 2

The anti-human mesothelin CAR-IL-7/CCL19-expressing T cells were found to exhibit excellent antitumor activity. In order to confirm longer-term antitumor effects and antitumor effects on cancer other than pancreatic cancer, a human malignant pleural mesothelioma cell line was administered to immunocompromised mice to form tumor. Then, the presence or absence of tumor recurrence for 143 days was examined with or without the administration of the anti-human mesothelin CAR-IL-7/CCL19-expressing T cells. The "method for producing an ACC-MESO-1-GFP-Luc line" and the "method for activating T cells" for use in this Example are as described below.

### (Production of ACC-MESO-1-GFP-Luc line)

A gene of green fluorescent protein-luciferase (GFP-Luc) was introduced using lentivirus to a human malignant mesothelioma cell line ACC-MESO-1, which is a mesothelin-positive tumor cell line kindly provided by Dr. Yoshitaka Sekido from Aichi Cancer Center Research Institute.

On day 0, ACC-MESO-1 was seeded at 1 × 10³ cells/well to a 96-well plate. The medium used was RPMI1640 (manufactured by Gibco/Thermo Fisher Scientific Inc.) supplemented with 10% FBS. On day 1, transduction was started by the addition of RediFect Red-FLuc-GFP (manufactured by PerkinElmer, Inc.), lentivirus particles for light emitting cell production, at MOI 100. In this respect, hexadimethrine bromide (manufactured by Sigma-Aldrich Co., LLC) was added at 4 µg/mL (final concentration) to the medium in order to enhance transfection efficiency. 24 hours after the virus addition (on day 2), the medium containing the virus was removed, followed by medium replacement. The culture was continued, and only cells expressing GFP were then sorted using SH800 (manufactured by Sony Corp.) to obtain ACC-MESO-1 expressing GFP, i.e., "ACC-MESO-1-GFP-Luc".

### (Production of anti-human mesothelin CAR-IL-7-CCL19-expressing T cell and anti-human mesothelin CAR-expressing T cell)

In this Example 7, the IL-7/CCL19 expression-anti-human mesothelin CAR vector (having the scFv region replaced with a VH07(15)VL07 DNA fragment, and signal peptide T shown in SEQ ID NO: 11 as the signal peptide) or the Conv. anti-human mesothelin CAR vector (having the scFv region replaced with a VH07(15)VL07 DNA fragment, and signal peptide T shown in SEQ ID NO: 11 as the signal peptide) obtained in Example 1 were used.

### (Activation of T cell)

On day 0, the culture of 2 × 10⁶ peripheral blood mononuclear cells collected from a healthy donor with IL-2 (manufactured by PeproTech, Inc.) was started at 37°C in a 5% CO₂ incubator on a 6-well plate for cell culture on which 25 µL/mL RetroNectin (manufactured by Takara Bio Inc.) and 5 µg/mL anti-human CD3 monoclonal antibody (manufactured by Invitrogen Corp.) were immobilized. The culture solution used was OpTmizer CTS (manufactured by Gibco/Thermo Fisher Scientific Inc.) supplemented with 2 mM L-glutamine (manufactured by Gibco/Thermo Fisher Scientific Inc.), 1% penicillin-streptomycin (manufactured by Wako Pure Chemical Industries, Ltd.) and 2.5 µg/mL Fungizone (manufactured by Bristol-Myers Squibb Company). The cells were cultured for 3 days. On day 3, morphological change in T cells caused by activation was confirmed under a microscope to obtain activated T cells.

### (Observation of tumor recurrence)

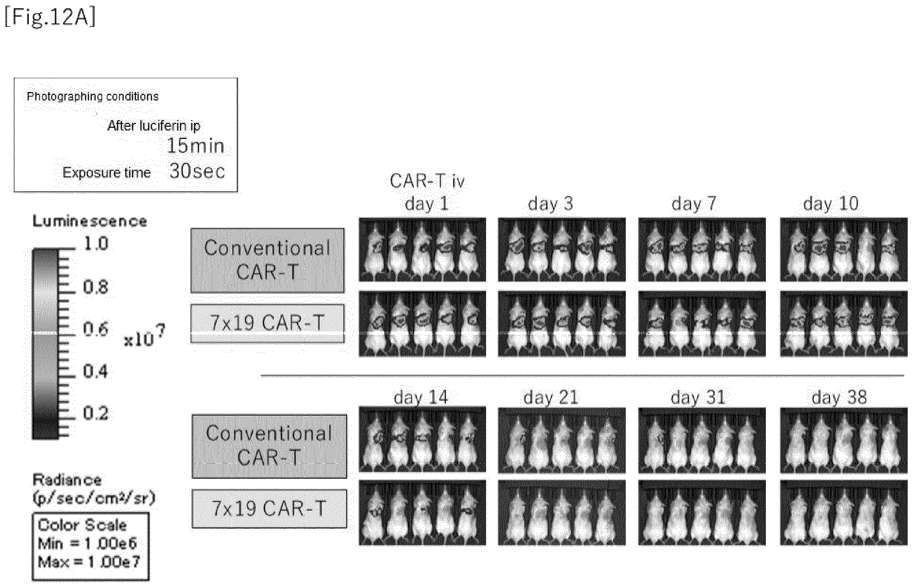

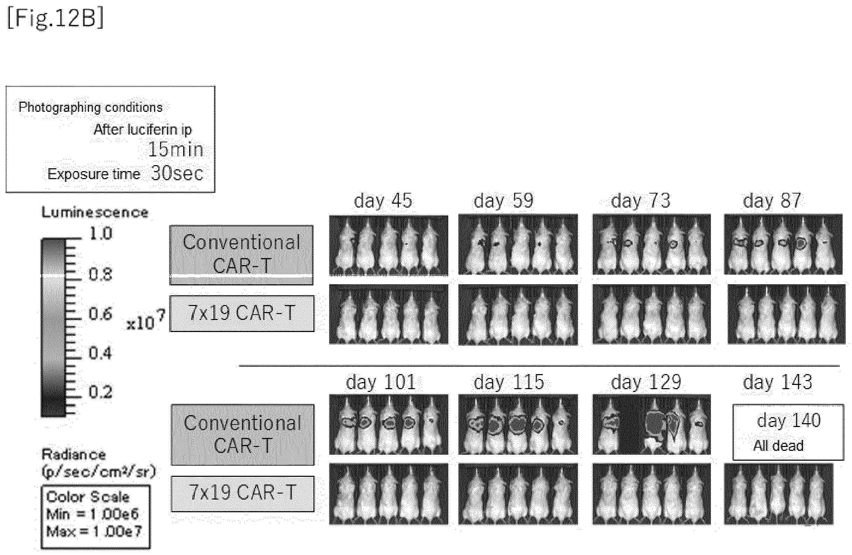

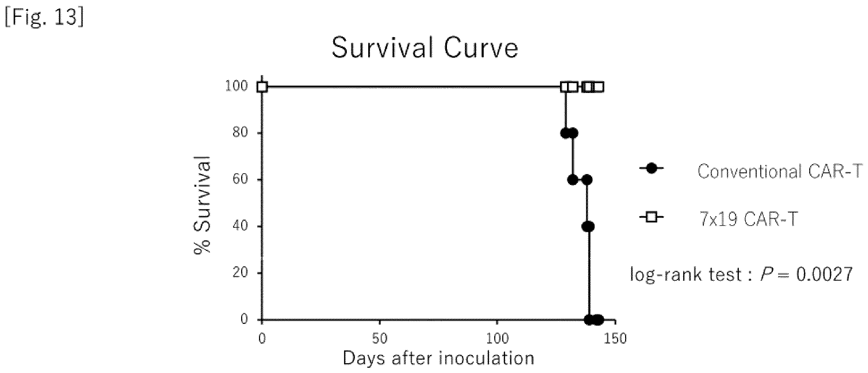

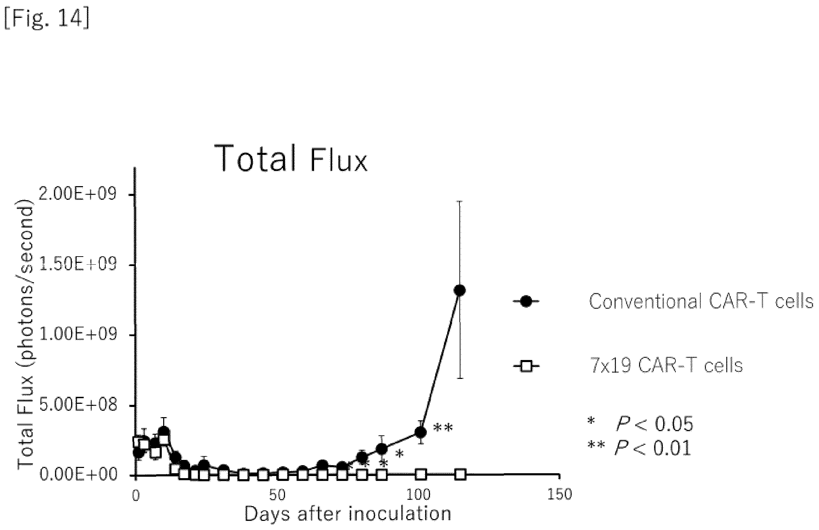

On day 0, first, the ACC-MESO-1-GFP-Luc was intrapleurally administered at 2 × 10⁶ cells/mouse to 8-week-old female NSG immunocompromised mice. On day 1, tumor engraftment in the pleural space was confirmed using an *in vivo* imaging system (IVIS). On day 1, the anti-human mesothelin CAR-expressing T cells and the anti-human mesothelin CAR-IL-7-CCL19-expressing T cells (scFv region: VH07(15)VL07) produced by the method of Example 1 and then frozen, and the T cells activated by the method described above were thawed. The anti-human mesothelin CAR-expressing T cells and the anti-human mesothelin CAR-IL-7-CCL19-expressing T cells had a CAR expression rate of 49.6% and 32.5%, respectively. Therefore, the activated T cells were added to the anti-human mesothelin CAR-expressing T cells to match their CAR expression rates. Then, a group given 1 × 10⁵ cells of the anti-human mesothelin CAR-expressing T cells (N = 5), and a group given 1 × 10⁵ cells of the anti-human mesothelin CAR-IL-7-CCL19-expressing T cells (N = 5) were provided. The administration of the anti-human mesothelin CAR-expressing T cells and the anti-human mesothelin CAR-IL-7-CCL19-expressing T cells was performed by intravenous administration from the tail veins. On day 3 and subsequent days, tumor fluorescence intensity was measured (total flux (photons/sec)) using IVIS. The results are shown in Figures 12A and 12B. The relationship between the number of days from administration and the survival rates of the mice in the results is shown in a graph form in Figure 13, and the relationship between the number of days from administration and the total quantity of fluorescence (photons/second) is shown in a graph form in Figure 14. In Figures 12A, 12B, 13, and 14, the mice given the anti-human mesothelin CAR-IL-7-CCL19-expressing T cells are shown by "7×19 CAR-T", and the mice given the anti-human mesothelin CAR-expressing T cells are shown by "Conventional CAR-T". In this Example 7, the influence of endogenous T cells of recipients was excluded because NSG immunocompromised mice deficient in endogenous T cells were used as the recipients. Thus, the effects of the administered anti-human mesothelin CAR-IL-7-CCL19-expressing T cells themselves were evaluated.

As shown in Figures 12A, 12B, 13, and 14, tumor fluorescence intensity was rarely observed in both 7×19 CAR-T and Conventional CAR-T on day 21. Tumor fluorescence was not observed in 7×19 CAR-T up to day 143, confirming that recurrence was completely suppressed. On the other hand, tumor fluorescence was observed in Conventional CAR-T from about day 45, and tumor fluorescence intensity elevated on day 115 with one mouse dead on day 129 and the remaining four mice dead on day 143. This demonstrated that the administered CAR-IL-7-CCL19-expressing T cells have cytotoxic activity not only against pancreatic cancer but against cancer (e.g., human malignant pleural mesothelioma) cells expressing human mesothelin, and have long-term antitumor effects.

## Claims

1. An immunocompetent cell that comprises: (a) an exogenous nucleic acid encoding a chimeric antigen receptor (CAR) specifically recognizing human mesothelin; (b) an exogenous nucleic acid encoding interleukin 7 (IL-7); and (c) an exogenous nucleic acid encoding chemokine (C-C motif) ligand 19 (CCL19),
the CAR comprising a single chain antibody, a transmembrane region, and a signaling region that induces activation of the immunocompetent cell,
wherein the single chain antibody in the CAR is (1-1) a single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18.

2. The immunocompetent cell according to claim 1, wherein the immunocompetent cell is an immunocompetent cell separated from a bodily fluid.

3. The immunocompetent cell according to claim 1, wherein the exogenous nucleic acid encoding IL-7, and the exogenous nucleic acid encoding CCL19 are an artificially synthesized nucleic acid encoding human IL-7, and an artificially synthesized nucleic acid encoding human CCL19.

4. The immunocompetent cell according to claim 3, wherein the exogenous nucleic acid encoding a CAR specifically recognizing human mesothelin, the exogenous nucleic acid encoding human IL-7, and the exogenous nucleic acid encoding human CCL19 are integrated into the genome.

5. The immunocompetent cell according to any one of claims 1 to 4, wherein the single chain antibody in the CAR is (1-3) a single chain antibody comprising a heavy chain variable region consisting of the amino acid sequence shown by SEQ ID NO: 1, and a light chain variable region consisting of the amino acid sequence shown by SEQ ID NO: 2.

6. The immunocompetent cell according to any one of claims 1 to 5, wherein the transmembrane region in the CAR comprises an amino acid sequence shown by SEQ ID NO: 7.

7. The immunocompetent cell according to any one of claims 1 to 6, wherein the signaling region that induces the activation of the immunocompetent cell in the CAR comprises at least one member selected from intracellular regions of polypeptides of CD28, 4-1BB (CD137), GITR, CD27, OX40, HVEM, CD3ζ, and Fc receptor-associated γ chain.

8. The immunocompetent cell according to any one of claims 1 to 6, wherein the signaling region that induces the activation of the immunocompetent cell in the CAR comprises the amino acid sequences shown by SEQ ID NOs: 8, 9 and 10.

9. The immunocompetent cell according to any one of claims 1 to 8, wherein the heavy chain variable region and the light chain variable region are connected via a peptide linker consisting of a 2- to 30-amino acid sequence.

10. The immunocompetent cell according to claim 9, wherein the peptide linker consists of the amino acid sequence shown by SEQ ID NO: 26 or SEQ ID NO: 27.

11. The immunocompetent cell according to any one of claims 1 to 10, wherein the signaling region that induces the activation of the immunocompetent cell in the CAR comprises a polypeptide of a 4-1BB intracellular region, a polypeptide of a CD3ζ intracellular region and a polypeptide of a CD28 intracellular region.

12. The immunocompetent cell according to any one of claims 1 to 11, wherein the immunocompetent cell is a T cell.

13. The immunocompetent cell according to any one of claims 1 to 12, wherein the immunocompetent cell is a T cell separated from a bodily fluid.

14. A pharmaceutical composition comprising an immunocompetent cell according to any one of claims 1 to 13 and a pharmaceutically acceptable additive.

15. The pharmaceutical composition according to claim 14 for use in the treatment of cancer.

16. An expression vector comprising a nucleic acid encoding a chimeric antigen receptor (CAR) specifically recognizing human mesothelin, a nucleic acid encoding IL-7, and a nucleic acid encoding CCL19, the CAR comprising a single chain antibody, a transmembrane region, and a signaling region that induces activation of an immunocompetent cell,
wherein the single chain antibody of the CAR is (1-1) a single chain antibody comprising a heavy chain variable region comprising heavy chain CDR1 consisting of the amino acid sequence shown by SEQ ID NO: 13, heavy chain CDR2 consisting of the amino acid sequence shown by SEQ ID NO: 14, and heavy chain CDR3 consisting of the amino acid sequence shown by SEQ ID NO: 15, and a light chain variable region comprising light chain CDR1 consisting of the amino acid sequence shown by SEQ ID NO: 16, light chain CDR2 consisting of the amino acid sequence shown by SEQ ID NO: 17, and light chain CDR3 consisting of the amino acid sequence shown by SEQ ID NO: 18.

17. A method for producing an immunocompetent cell that expresses a chimeric antigen receptor (CAR) specifically recognizing human mesothelin, IL-7, and CCL19, comprising introducing the expression vector of claim 16 to an immunocompetent cell.

## Patentansprüche

1. Immunkompetente Zelle, die Folgendes umfasst: (a) eine exogene Nukleinsäure, die einen chimären Antigenrezeptor (CAR) kodiert, der spezifisch humanes Mesothelin erkennt; (b) eine exogene Nukleinsäure, die Interleukin 7 (IL-7) kodiert; und (c) eine exogene Nukleinsäure, die den Chemokin-(C-C Motiv-)Liganden 19 (CCL19) kodiert, wobei der CAR einen Einzelketten-Antikörper, eine Transmembranregion und eine Signalregion, die eine Aktivierung der immunkompetenten Zelle hervorruft, umfasst, wobei der Einzelketten-Antikörper in dem CAR (1-1) ein Einzelketten-Antikörper ist, umfassend eine variable Region der schweren Kette, umfassend eine CDR1 der schweren Kette, die aus der von SEQ ID Nr. 13 gezeigten Aminosäuresequenz besteht, eine CDR2 der schweren Kette, die aus der von SEQ ID Nr. 14 gezeigten Aminosäuresequenz besteht und eine CDR3 der schweren Kette, die aus der von SEQ ID Nr. 15 gezeigten Aminosäuresequenz besteht, und eine variable Region der leichten Kette, umfassend eine CDR1 der leichten Kette, die aus der von SEQ ID Nr. 16 gezeigten Aminosäuresequenz besteht, eine CDR2 der leichten Kette, die aus der von SEQ ID Nr. 17 gezeigten Aminosäuresequenz besteht und eine CDR3 der leichten Kette, die aus der von SEQ ID Nr. 18 gezeigten Aminosäuresequenz besteht.

2. Immunkompetente Zelle nach Anspruch 1, wobei die immunkompetente Zelle eine immunkompetente Zelle ist, die von einer Körperflüssigkeit getrennt ist.

3. Immunkompetente Zelle nach Anspruch 1, wobei die exogene Nukleinsäure, die IL-7 kodiert, und die exogene Nukleinsäure, die CCL19 kodiert, eine künstlich synthetisierte Nukleinsäure, die humanes IL-7 kodiert und eine künstlich synthetisierte Nukleinsäure, die humanes CCL19 kodiert, sind.

4. Immunkompetente Zelle nach Anspruch 3, wobei die exogene Nukleinsäure, die einen CAR kodiert, der spezifisch humanes Mesothelin kodiert, die exogene Nukleinsäure, die humanes IL-7 kodiert und die exogene Nukleinsäure, die humanes CCL19 kodiert, in das Genom integriert sind.

5. Immunkompetente Zelle nach einem der Ansprüche 1 bis 4, wobei der Einzelketten-Antikörper in dem CAR (1-3) ein Einzelketten-Antikörper ist, umfassend eine variable Region der schweren Kette, die aus der von SEQ ID Nr. 1 gezeigten Aminosäuresequenz besteht, und eine variable Region der leichten Kette, die aus der von SEQ ID Nr. 2 gezeigten Aminosäuresequenz besteht.

6. Immunkompetente Zelle nach einem der Ansprüche 1 bis 5, wobei die Transmembranregion in dem CAR eine von SEQ ID Nr. 7 gezeigte Aminosäuresequenz umfasst.

7. Immunkompetente Zelle nach einem der Ansprüche 1 bis 6, wobei die Signalregion, die die Aktivierung der immunkompetenten Zelle in dem CAR hervorruft, mindestens ein Mitglied, das ausgewählt ist aus intrazellulären Regionen von Polypeptiden von CD28, 4-1BB (CD137), GITR, CD27, OX40, HVEM, CD3ζ und eine Fc Rezeptor-assoziierte γ-Kette umfasst.

8. Immunkompetente Zelle nach einem der Ansprüche 1 bis 6, wobei die Signalregion, die die Aktivierung der immunkompetenten Zelle in dem CAR hervorruft, die von SEQ ID Nr. 8, 9 und 10 gezeigte Aminosäuresequenz umfasst.

9. Immunkompetente Zelle nach einem der Ansprüche 1 bis 8, wobei die variable Region der schweren Kette und die variable Region der leichten Kette über einen Peptid-Linker verbunden sind, der aus einer 2- bis 30-Aminosäuresequenz besteht.

10. Immunkompetente Zelle nach Anspruch 9, wobei der Peptid-Linker aus der von SEQ ID Nr. 26 oder SEQ ID Nr. 27 gezeigten Aminosäuresequenz besteht.

11. Immunkompetente Zelle nach einem der Ansprüche 1 bis 10, wobei die Signalregion, die die Aktivierung der immunkompetenten Zelle in dem CAR hervorruft, ein Polypeptid einer 4-1BB intrazellulären Region, ein Polypeptid einer CD3ζ intrazellulären Region und ein Polypeptid einer CD28 intrazellulären Region umfasst.

12. Immunkompetente Zelle nach einem der Ansprüche 1 bis 11, wobei die immunkompetente Zelle eine T-Zelle ist.

13. Immunkompetente Zelle nach einem der Ansprüche 1 bis 12, wobei die immunkompetente Zelle eine T-Zelle ist, die von einer Körperflüssigkeit getrennt ist.

14. Pharmazeutische Zusammensetzung, umfassend eine immunkompetente Zelle nach einem der Ansprüche 1 bis 13 und ein pharmazeutisch verträgliches Additiv.

15. Pharmazeutische Zusammensetzung nach Anspruch 14 zur Verwendung in der Behandlung von Krebs.

16. Expressionsvektor, umfassend eine Nukleinsäure, die einen chimären Antigenrezeptor (CAR) kodiert, der spezifisch humanes Mesothelin erkennt, eine Nukleinsäure, die IL-7 kodiert, und eine Nukleinsäure, die CCL19 kodiert, wobei der CAR einen Einzelketten-Antikörper, eine Transmembranregion und eine Signalregion, die eine Aktivierung einer immunkompetenten Zelle hervorruft, umfasst,
wobei der Einzelketten-Antikörper in dem CAR (1-1) ein Einzelketten-Antikörper ist, umfassend eine variable Region der schweren Kette, umfassend eine CDR1 der schweren Kette, die aus der von SEQ ID Nr. 13 gezeigten Aminosäuresequenz besteht, eine CDR2 der schweren Kette, die aus der von SEQ ID Nr. 14 gezeigten Aminosäuresequenz besteht und eine CDR3 der schweren Kette, die aus der von SEQ ID Nr. 15 gezeigten Aminosäuresequenz besteht; und eine variable Region der leichten Kette, umfassend eine CDR1 der leichten Kette, die aus der von SEQ ID Nr. 16 gezeigten Aminosäuresequenz besteht, eine CDR2 der leichten Kette, die aus der von SEQ ID Nr. 17 gezeigten Aminosäuresequenz besteht und eine CDR3 der leichten Kette, die aus der von SEQ ID Nr. 18 gezeigten Aminosäuresequenz besteht.

17. Verfahren zum Herstellen einer immunkompetenten Zelle, die einen chimären Antigenrezeptor (CAR) exprimiert, der spezifisch humanes Mesothelin, IL-7 und CCL19 erkennt, umfassend das Einführen des Expressionsvektors von Anspruch 16 in eine immunkompetente Zelle.

## Revendications

1. Cellule immunocompétente qui comprend : (a) un acide nucléique exogène codant pour un récepteur antigénique chimérique (CAR) reconnaissant spécifiquement la mésothéline humaine ; (b) un acide nucléique exogène codant pour l'interleukine 7 (IL-7) ; et (c) un acide nucléique exogène codant pour le ligand 19 (CCL19) de la chimiokine (motif C-C), le CAR comprenant un anticorps monocaténaire, une région transmembranaire et une région de signalisation qui induit l'activation de la cellule immunocompétente,
dans lequel l'anticorps monocaténaire dans le CAR est (1-1) un anticorps monocaténaire comprenant une région variable de chaîne lourde comprenant la chaîne lourde CDR1 consistant en la séquence d'acides aminés représentée par la SEQ ID NO : 13, la chaîne lourde CDR2 consistant en la séquence d'acides aminés représentée par la SEQ ID NO : 14 et la chaîne lourde CDR3 consistant en la séquence d'acides aminés représentée par la SEQ ID NO : 15, et une région variable de chaîne légère comprenant la chaîne légère CDR1 consistant en la séquence d'acides aminés représentée par la SEQ ID NO : 16, la chaîne légère CDR2 consistant en la séquence d'acides aminés représentée par la SEQ ID NO: 17 et la chaîne légère CDR3 consistant en la séquence d'acides aminés représentée par la SEQ ID NO : 18.

2. Cellule immunocompétente selon la revendication 1, dans laquelle la cellule immunocompétente est une cellule immunocompétente séparée d'un liquide corporel.

3. Cellule immunocompétente selon la revendication 1, dans laquelle l'acide nucléique exogène codant pour l'IL-7 et l'acide nucléique exogène codant pour le CCL19 sont un acide nucléique synthétisé artificiellement codant pour l'IL-7 humain et un acide nucléique synthétisé artificiellement codant pour le CCL19 humain.

4. Cellule immunocompétente selon la revendication 3, dans laquelle l'acide nucléique exogène codant pour un CAR reconnaissant spécifiquement la mésothéline humaine, l'acide nucléique exogène codant pour l'IL-7 humaine et l'acide nucléique exogène codant pour le CCL19 humain sont intégrés au génome.

5. Cellule immunocompétente selon l'une quelconque des revendications 1 à 4, dans laquelle l'anticorps monocaténaire dans le CAR est (1-3) un anticorps monocaténaire comprenant une région variable de chaîne lourde consistant en la séquence d'acides aminés représentée par la SEQ ID NO : 1, et une région variable de chaîne légère consistant en la séquence d'acides aminés représentée par la SEQ ID NO : 2.

6. Cellule immunocompétente selon l'une quelconque des revendications 1 à 5, dans laquelle la région transmembranaire dans le CAR comprend une séquence d'acides aminés représentée par la SEQ ID NO : 7.

7. Cellule immunocompétente selon l'une quelconque des revendications 1 à 6, dans laquelle la région de signalisation qui induit l'activation de la cellule immunocompétente dans le CAR comprend au moins un élément choisi parmi les régions intracellulaires des polypeptides de CD28, 4-1BB (CD137), GITR, CD27, OX40, HVEM, CD3ζ, et la chaîne γ associée au récepteur Fc.

8. Cellule immunocompétente selon l'une quelconque des revendications 1 à 6, dans laquelle la région de signalisation qui induit l'activation de la cellule immunocompétente dans le CAR comprend les séquences d'acides aminés représentée par les SEQ ID NOs : 8, 9 et 10.

9. Cellule immunocompétente selon l'une quelconque des revendications 1 à 8, dans laquelle la région variable de chaîne lourde et la région variable de chaîne légère sont reliées par un lieur peptidique consistant en une séquence d'acides aminés de 2 à 30.

10. Cellule immunocompétente selon la revendication 9, dans laquelle le lieur peptidique consistant en la séquence d'acides aminés représentée par la SEQ ID NO : 26 ou la SEQ ID NO : 27.

11. Cellule immunocompétente selon l'une quelconque des revendications 1 à 10, dans laquelle la région de signalisation qui induit l'activation de la cellule immunocompétente dans le CAR comprend un polypeptide d'une région intracellulaire 4-1BB, un polypeptide d'une région intracellulaire CD3ζ et un polypeptide d'une région intracellulaire CD28.

12. Cellule immunocompétente selon l'une quelconque des revendications 1 à 11, dans laquelle la cellule immunocompétente est un lymphocyte T.

13. Cellule immunocompétente selon l'une quelconque des revendications 1 à 12, dans laquelle la cellule immunocompétente est un lymphocyte T séparé d'un fluide corporel.

14. Composition pharmaceutique comprenant une cellule immunocompétente selon l'une quelconque des revendications 1 à 13 et un additif pharmaceutiquement acceptable.

15. Composition pharmaceutique selon la revendication 14 pour une utilisation dans le traitement du cancer.

16. Vecteur d'expression comprenant un acide nucléique codant pour un récepteur antigénique chimérique (CAR) reconnaissant spécifiquement la mésothéline humaine, un acide nucléique codant pour l'IL-7 et un acide nucléique codant pour le CCL19, le CAR comprenant un anticorps monocaténaire, une région transmembranaire, et une région de signalisation qui induit l'activation d'une cellule immunocompétente,
dans lequel l'anticorps monocaténaire du CAR est (1-1) un anticorps monocaténaire comprenant une région variable de chaîne lourde comprenant la chaîne lourde CDR1 consistant en la séquence d'acides aminés représentée par la SEQ ID NO : 13, la chaîne lourde CDR2 consistant en la séquence d'acides aminés représentée par la SEQ ID NO : 14 et la chaîne lourde CDR3 consistant en la séquence d'acides aminés représentée par SEQ ID NO : 15, et une région variable de chaîne légère comprenant la chaîne légère CDR1 consistant en la séquence d'acides aminés représentée par la SEQ ID NO : 16, la chaîne légère CDR2 consistant en la séquence d'acides aminés représentée par la SEQ ID NO : 17 et la chaîne légère CDR3 consistant en la séquence d'acides aminés représentée par la SEQ ID NO : 18.

17. Procédé de production d'une cellule immunocompétente qui exprime un récepteur antigénique chimérique (CAR) reconnaissant spécifiquement la mésothéline humaine, l'IL-7 et le CCL19, comprenant l'introduction du vecteur d'expression selon la revendication 16 dans une cellule immunocompétente.
